(19) Europäisches Patentamt
European Patent Office
Office européen des brevets

(11) EP 3 558 361 B1

(12) EUROPEAN PATENT SPECIFICATION

(45) Date of publication and mention
of the grant of the patent:
**20.08.2025 Bulletin 2025/34**

(21) Application number: **17829315.5**

(22) Date of filing: **20.12.2017**

(51) International Patent Classification (IPC):
**A61K 39/395** (2006.01)    **A61K 45/00** (2006.01)
**A61P 3/04** (2006.01)

(52) Cooperative Patent Classification (CPC):
**C07K 16/2863; A61P 3/00; A61P 3/04; A61P 5/00;**
A61K 2039/505; A61K 2039/545; C07K 2317/21;
C07K 2317/76

(86) International application number:
**PCT/IB2017/058199**

(87) International publication number:
**WO 2018/116201 (28.06.2018 Gazette 2018/26)**

(54) **MYOSTATIN, ACTIVIN OR ACTIVIN RECEPTOR ANTAGONISTS FOR USE IN TREATING OBESITY AND RELATED CONDITIONS**

ANTAGONISTEN VON MYOSTATIN, AKTIVIN ODER AKTIVINREZEPTOREN ZUR VERWENDUNG IN DER BEHANDLUNG VON OBESITÄT UND VERWANDTEN KRANKHEITEN

ANTAGONISTES DE LA MYOSTATINE, DE L'ACTIVINE OU DES RÉCEPTEURS ACTIVINE POUR L'UTILISATION DANS LE TRAITEMENT DE L'OBÉSITÉ ET MALADIES ASSOCIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **21.12.2016 US 201662437097 P**

(43) Date of publication of application:
**30.10.2019 Bulletin 2019/44**

(73) Proprietor: **Novartis AG**
**4056 Basel (CH)**

(72) Inventors:
• **GARITO, Tania Silvia**
**4002 Basel (CH)**
• **ROUBENOFF, Ronenn**
**4002 Basel (CH)**
• **ZAKARIA, Marjorie**
**Cambridge, MA 02139 (US)**

(74) Representative: **Cooley (UK) LLP**
**22 Bishopsgate**
**London EC2N 4BQ (GB)**

(56) References cited:
**WO-A1-2010/144452    WO-A1-2016/205370**

• **DATABASE BIOSIS [online] BIOSCIENCES INFORMATION SERVICE, PHILADELPHIA, PA, US; March 2011 (2011-03-01), ATTIE KENNETH M ET AL: "Increased Lean Mass and Muscle Volume in Healthy Post-Menopausal Women Treated with ACE-031 (Soluble Activin Type IIB Receptor), an Inhibitor of Myostatin and Other Negative Regulators of Muscle", Database accession no. PREV201100271069**
• **NEUROLOGY, vol. 76, no. 9, Suppl. 4, March 2011 (2011-03-01), 63RD ANNUAL MEETING OF THE AMERICAN-ACADEMY-OF-NEUROLOGY; HONOLULU, HI, USA; APRIL 09 -16, 2011, pages A281 - A282, ISSN: 0028-3878**

- **ATTIE KENNETH M ET AL: "Increased Lean Mass and Muscle Volume in Healthy Post-Menopausal Women Treated with ACE-031 (Soluble Activin Type IIB Receptor), an Inhibitor of Myostatin and Other Negative Regulators of Muscle", NEUROLOGY, vol. 76, no. 9, Suppl. 4, March 2011 (2011-03-01), & 63RD ANNUAL MEETING OF THE AMERICAN-ACADEMY-OF-NEUROLOGY; HONOLULU, HI, USA; APRIL 09 -16, 2011, pages A281 - A282, XP002778723, ISSN: 0028-3878**
- **ASHTON R. ET AL.: "The effects of a natural myostatin inhibitor derived from fertilized egg yolk on serum myostatin level and lean muscle thickness and mass: A randomized, double-blind, placebo-controlled trial", THE JOURNAL OF FRAILTY & AGING, vol. 4, no. Suppl.1, 2015, pages 74, XP002778724**
- **TANKÓ LÁSZLÓ B ET AL: "Does Activin Receptor Blockade by Bimagrumab (BYM338) Pose Detrimental Effects on Bone Healing in a Rat Fibula Osteotomy Model?", CALCIFIED TISSUE INTERNATIONAL, NEW YORK, NY, US, vol. 99, no. 3, 11 May 2016 (2016-05-11), pages 310 - 321, XP036014491, ISSN: 0171-967X, [retrieved on 20160511], DOI: 10.1007/ S00223-016-0148-0**

Remarks:

The complete document including Reference Table(s) and the Sequence Listing(s) can be downloaded from the EPO website

**Description**

FIELD OF THE INVENTION

The present invention relates to specific anti-ActRII receptor antibodies for use in the treatment of obesity or overweight in human subjects and for use in improving glycemic control in human subjects suffering from type II diabetes.

BACKGROUND OF THE INVENTION

In 2014, more than 1.9 billion adults worldwide (39%) were estimated to be overweight; of those, 600 million (13%) were obese (World Health Organization 2015). Recent estimates indicate that roughly 78 million adults in the United States are obese (Jensen et al 2014), 69% are either overweight or obese and 35% are obese (Ogden et al 2014). Obesity is a risk factor of overall mortality and is estimated to have caused 3.4 million deaths worldwide in 2010 (Lim et al 2012). Many co-morbidities are associated with obesity, such as type 2 diabetes, hypertension, dyslipidemia, coronary heart disease (Apovian et al 2015). Insulin resistance is a condition of tolerance to insulin, making the hormone less effective, causing decreased glucose uptake in muscle tissue that result in impaired glucose oxidation and glycogen synthesis, and a deficient suppression of hepatic glucose production in the liver. In obese, increased visceral fat mass with elevation of plasma free fatty acid (FFA) caused by the intensified lipolytic activity, worsen insulin resistance through the impairment of insulin action (Reaven et al., 1988); a mechanism known as lipotoxicity (DeFronzo, 2004). High concentrations of FFA in skeletal muscle cells lead to reduction in insulin-stimulated intracellular transport of glucose through the Glut4 transporter (Dresner et al., 1999), in the hepatocytes lead to enhanced rate of gluconeogenesis and glucose release from the liver and augmented insulin secretion from β cells in response to a transient increase of FFA or an inhibition effect in response to chronic elevated levels (Boden, 1997). As a result of insulin resistance and lipotoxicity, more insulin is needed to induce glucose uptake from fat and muscle cells, and glycogen synthesis in the liver (Cefalu et al., 2016). The overproduction of insulin by pancreatic β cells is the physiologic reaction to insulin resistance and can lead to decline of β cell function and, eventually to prediabetes and type 2 diabetes (Donath et al., 2005). Genetic predisposition, age and lifestyle (overweight/obesity and inactivity) are risk factors for insulin resistance. Moreover, insulin resistance associated with type 2 diabetes is responsible for inadequate glycemic control, leading to failure of oral hypoglycemic agents (OHA) and, eventually, the need to initiate insulin therapy.

A body weight reduction of 5-10% has been associated with significant and clinically meaningful improvements in insulin sensitivity, glycemic control, hypertension and dyslipidemia.

Lifestyle changes through dietary counseling and regular physical activity are the first steps toward weight loss and glycemic control (Cefalu et al., 2016). Thus, aerobic exercise training improves insulin sensitivity in insulin-resistant patients by increasing glycogen synthesis in muscles through the increase in glucose transport-phosphorylation (Glut4) stimulated by insulin (Perseghin et al., 1996).

Obesity treatment is challenging. Lifestyle interventions are effective in the setting of clinical trials with intensive counseling, but not as effective in real life due to difficulty in adherence and maintenance. Obesity pharmacotherapy is limited, associated with side effects and results in loss of both fat body mass (FBM) and lean body mass (LBM) (Heymsfield et al 2014). Weight loss studies have consistently shown loss of lean tissue on the order of ~ 1/4th of loss of body composition. A meta-analysis of 26 overweight or obese cohorts with dietary and behavioral interventions reported a mean weight loss >10 kg. The analysis showed that the magnitude of caloric restriction was significantly (p=0.006) associated with the percent of weight loss as lean mass. The ratio of change in lean mass over body weight loss was greater (p=0.08) in men (X±SD; 27±7%) compared to women (20±7%) (Chaston et al 2007). For example, for every 1 kg of fat mass lost during weight loss, approximately 0.26 kg of lean tissue was lost in post-menopausal women (Chmelo et al 2016). Importantly, the loss of lean mass is only partially regained with weight gain, i.e. for every 1 kg of fat mass regained in the following year, only 0.12 kg of lean tissue was regained indicating that there is a persistent negative balance on lean tissue (Chmelo et al 2016). Other studies have shown that with weight regain, about one-half (45%) of total fat mass had been regained but lost lean mass was not recovered. Obesity trials that reported effects on body composition included exercise in the form aerobic activities and an observed reduction in fat free mass losses of about one-half that of diet-only groups (Heymsfield et al 2014), indicating that the addition of an exercise regimen can partially protect against lean mass loss. There are no approved obesity interventions that preserve or build lean mass while promoting fat mass loss.

The Activin type 2 receptors (ActRIIA and ActRIIB, collectively abbreviated as ActRII) modulate signals for ligands belonging to the transforming growth factor beta (TGF-β) superfamily such as myostatin, GDF-11, and activins. Myostatin, activin A, and GDF-11 are negative regulators of skeletal muscle growth, acting via the ActRII receptor signaling pathway to inhibit muscle protein synthesis and myocyte differentiation and proliferation. Preclinical reports, in normal or high fat fed mice, indicate that the increase in muscle mass, observed with either myostatin knockout mice or sequestration of myostatin by soluble ActRIIB, is associated with improved whole body insulin sensitivity, measured by glucose and insulin tolerance tests and by hyperinsulinemic-euglycemic clamp, and with resistance to diet-induced and genetic obesity (Guo et al, 2009, Akpan et al, 2009).

Bimagrumab (BYM338), a recombinant human, monoclonal antibody binds competitively to ActRII with greater affinity than its natural ligands. Bimagrumab is in development for muscle wasting indications and has shown a significant increase in skeletal muscle mass in healthy volunteers, in patients with sporadic inclusion body myositis (sIBM), and in patients with sarcopenia. In previous studies, a single dose of bimagrumab caused an increase in thigh muscle volume measured by magnetic resonance imaging of approximately 6% after 10 weeks in healthy lean adults compared to placebo, and reduced fat mass to a comparable extent (Roubenoff and Papanicolaou, New treatments for muscle wasting: an update on bimagrumab and other treatments. Abstract at ICFSR 2015). A single dose of bimagrumab resulted in a profound impact on body composition with a maximal reduction in fat mass of ~ 8% and an increase in lean mass of ~ 3% (DXA), in overweight/obese pre-diabetic patients (Garito T, Diabetes Obesity and Metabolism, 2017). The net effect on total body weight was neutral. Insulin sensitivity was improved by a ~ 18% as measured with a two-step hyperinsulinemic euglycemic clamp. This effect was associated with an absolute reduction in HbA1c of 0.2%, an effect size which has been associated with prevention of progression to diabetes in a similar population (DeFronzo et al 2011, Knowler et al 2002). In patients affected by sporadic inclusion body myositis (sIBM), 10 mg/kg of bimagrumab for 1 year every 4 weeks i.v. for a total of 12 doses, resulted in a progressive decrease in body weight: at week 52 patients who received bimagrumab 10 mg/kg monthly lost on average 3.68 kg, compared to 270 grams in patients treated with placebo; a gain of lean mass on average of 1.6 kg, compared to a loss of 750 grams in the placebo treated group; a loss on average of 5.05 kg of fat body mass, compared to 120 grams in patients treated with placebo (data not published BYM338B2203)

WO2013/006437 (Novartis AG) relates to the treatment of metabolic disorders including obesity but it relates the increase of brown adipose tissue and its thermogenic activity as mechanism to reduce fat, but not to loss of fat (white adipose tissue) combined with the increase in lean mass. In BYM338X2206, there was a trend toward a beneficial effect of ActRIIB blockade on BAT activity, although not statistically significant; this may be the result of an activation of mitochondrial oxidative metabolism in BAT as supported by preclinical data [Fournier et al., 2012]. Interestingly, preclinical studies have shown that not only are intracellular triglycerides the main fuel to sustain BAT energy metabolism, but a cold challenge may also result in a near complete depletion of BAT lipids [Ouellet et al., 2012]. This mechanism may then underlie the shrinkage of BAT volume which was also observed in this study in response to the bimagrumab treatment. The lack of significant results in BAT mass and activity in BYM338X2206 determined the interruption of investigations on this pathway.

WO 2010/144452 discloses the use of ActRIIB antagonists, in particular ActRIIB fusion proteins, for the treatment of obesity and metabolic diseases such as type 2 diabetes.

Treatment of obesity or overweight condition and their related comorbidities, in particular type II diabetes represent a substantial, unmet medical need. Therefore, pharmacotherapeutics that can improve body composition, by changing the ratio of lean mass versus fat mass and thereby achieve weight loss or decrease of central adiposity, and improve patient glycemic status are highly desired.

The current treatment is designed in obese and overweight individuals with type 2 diabetes to evaluate the effect of bimagrumab on body composition and the metabolic impact of these changes on glycemic parameters. The intent is to evaluate whether loss of fat mass along with increased lean mass, i.e. body weight neutral intervention, favorably improves insulin sensitivity and glycemic parameters, e.g. HbA1c, thereby representing a model for strategies to target fat loss, such as central adiposity (abdominal or truncal adiposity), in obese, overweight or even in persons with normal BMI.

## SUMMARY OF THE INVENTION

The present invention is defined in the appended claims. It is based on the therapeutic approach that sufficiently blocking myostatin or activin binding to their receptors ActRII (preferably ActRIIB and ActRIIA, or ActRIIA or ActRIIB either alone) significantly reduces the activity of myostatin and other ligands that inhibit skeletal muscle growth acting at the receptors, while allowing some of those ligands to perform other physiologic functions via alternative type II receptors (Upton et al 2009). Other approaches to reducing myostatin activity, i.e. competitive soluble ActRII, creating a soluble receptor sink may deplete a range of ActRII ligands with activities at other receptors, potentially creating a greater safety risk than using a receptor antagonist antibody like bimagrumab.

As a potent inhibitor of ActRII, bimagrumab blocks the effects of myostatin, activin A, GDF11, and possibly other ligands working through those receptors.

The present disclosure therefore provides a myostatin or activin antagonist or receptor antagonist, preferably a myostatin binding molecule or antibody, and more preferably an anti-ActRII receptor antibody, most preferably bimagrumab, for use in improving body composition.

The present disclosure therefore provides a myostatin or activin antagonist or receptor antagonist, preferably a myostatin binding molecule or antibody, and more preferably an anti-ActRII receptor antibody, most preferably bimagrumab, for use in the treatment of obesity or overweight condition.

In a similar aspect the present disclosure provides a myostatin or activin receptor antagonist, preferably a myostatin receptor antagonist, preferably a myostatin or activin binding molecule or antibody, and even more preferably an anti-ActRII receptor antibody, most preferably bimagrumab, for use in the treatment of obesity in a patient by improving body composition, whereby lean mass is increased, and fat mass is reduced.

In a similar aspect the present invention provides an activin antagonist, which is an anti-ActRII receptor antibody, most preferably bimagrumab, for use in the treatment, prevention or reduction of obesity or overweight condition related comorbidities.

In a further aspect, the present invention provides an activin antagonist, which is an anti-ActRII receptor antibody, most preferably bimagrumab, for use in improving glycemic control in a patient suffering from type II diabetes.

The present invention further provides specific dose regimen for the myostatin receptor antagonist bimagrumab for use herein.

BRIEF DESCRIPTION OF THE DRAWINGS

In the following the present invention is described in detail with reference to accompanying figures in which:

**Figure 1.** Body composition change from baseline, assessed by DXA. Data are presented as arithmetic mean (SE). A: Lean body mass. B: Fat body mass.

**Figure 2.** Insulin concentration vs. time for IVGTT by visit and treatment group. Data are presented as arithmetic mean (SE)

**Figure 3.** Individual percent change from baseline fat body mass by DXA *vs.* M/I at Step 1 and 2. Panel A and B show a linear and significant relationship between loss of body fat mass and increase in insulin sensitivity for Step 1 and Step 2. Individual percent change from baseline lean body mass by DXA *vs.* M/I at Step 1 and 2. It does not appear to be a relationship between changes in lean body mass and insulin sensitivity, both at Step 1 (C) and 2 (D).

**Figure 4.** $HbA_{1c}$ absolute change from baseline vs. time. Data presented as arithmetic mean (SE)

**Figure 5.** Arithmetic mean (SD) concentration-time plot by treatment. The pharmacokinetic analysis shows a non linear clearance profile.

**Figure 6** shows the study design.

DETAILED DESCRIPTION OF THE INVENTION

Herein after, the present invention is described in further detail and is exemplified.

The present disclosure provides the following aspects:

1. A myostatin or activin or activin receptor antagonist for use in the improvement of body composition in a human subject, whereby lean mass is increased, and fat mass is reduced.

2. A myostatin or activin or activin receptor antagonist for use in the treatment of central adiposity in a human subject, wherein said subject is obese, overweight or of normal BMI.

3. A myostatin or activin or activin receptor antagonist for use according to embodiment 1 or 2, wherein said patient has a BMI $\geq$ 30 kg/m$^2$.

4. A myostatin or activin or activin receptor antagonist for use according to embodiment 1 or 2, wherein said patient has a BMI $\geq$ 25 and < 30 kg/m$^2$.

5. A myostatin or activin or activin receptor antagonist for use according to embodiment 1 or 2, wherein said patient has a BMI < 25.

6. A myostatin or activin or activin receptor antagonist for use according to any of embodiments 1-5, wherein central adiposity is reduced.

7. A myostatin or activin or activin receptor antagonist for use in the treatment, prevention or reduction of obesity or overweight condition and related comorbidities.

8. A myostatin or activin or activin receptor antagonist for use according to embodiment 7 wherein obesity or overweight condition-related comorbidities are selected from the group of type 2 diabetes, glucose intolerance, prediabetes, insulin resistance, high triglycerides, physical impairment, osteoporosis , renal disease, obstructive sleep apnea, sexual hormones impairment, endocrine reproductive disorders such as polycystic ovary syndrome or male hypogonadism, osteoarthritis, gastrointestinal cancers, dyslipidaemia, hypertension, heart failure, coronary heart disease, stroke, gallstones, altered gonadal hormone profile.

9. A myostatin or activin or activin receptor antagonist for use according to embodiments 7-8 for the treatment, reduction or prevention of type II diabetes .

10. A myostatin or activin or activin receptor antagonist for use according to embodiments 7-9, wherein improvement of body composition is comprising reduction of central adiposity.

11. A myostatin or activin or activin receptor antagonist for use according to embodiment 10 for the prevention of type II diabetes and treatment of obesity or overweight condition.

12. A myostatin or activin or activin receptor antagonist for use according to embodiment 11 antagonist for use in improving glycemic control.

13. A myostatin or activin or activin receptor antagonist for use according to embodiment 12 wherein improving glycemic control is achieved by improving insulin sensitivity.

14. A myostatin or activin or activin receptor antagonist for use in improving glycemic control in a patient suffering from type II diabetes.

15. A myostatin or activin or activin receptor antagonist for use according to embodiment 14 wherein improving glycemic control is achieved by improving insulin sensitivity.

16. A myostatin or activin or activin receptor antagonist for use according to embodiments 14-15 wherein the patient is suffering from obesity or overweight condition.

17. A myostatin or activin or activin receptor antagonist for use according to embodiment 16 wherein DTSQ score (Diabetes treatment satisfaction questionnaire score, or IWQOL/IWQOL-lite (Impact of Weight on Quality of Life) score or other PRO related to obesity and/or diabetes are improved.

18. A myostatin or activin or activin receptor antagonist for use according to any preceding embodiment wherein the myostatin or activin antagonist is an anti-ActRII receptor antibody.

19. A myostatin or activin or activin receptor antagonist for use according to embodiment 18 wherein the anti-ActRII receptor antibody is bimagrumab.

20. A myostatin or activin or activin receptor antagonist for use according to embodiment 19, wherein bimagrumab is to be administered at a dose of 3 mg/kg or 10 mg/kg.

21. A myostatin or activin or activin receptor antagonist for use according to embodiment 20, wherein bimagrumab is to be administered every 4 weeks.

22. A method of treatment of improving body composition in a patient in need thereof whereby lean mass is increased and fat mass is reduced, comprising administering a myostatin or activin antagonist or activin receptor antagonist.

23. A method of treatment of central adiposity in a patient in need thereof, wherein said subject is obese, overweight or of normal BMI comprising administering a myostatin or activin antagonist or activin receptor antagonist.

24. A method of treatment according to embodiment 22 or 23, wherein said patient has a BMI $\geq 30$ kg/m$^2$.

25. A method of treatment according to embodiment 22 or 23, wherein said patient has a BMI $\geq 25 - < 30$ kg/m$^2$.

26. A method of treatment according to embodiment 22 or 23, wherein said patient has a BMI < 25.

27. A method of treatment according to any of embodiments 22-26, wherein central adiposity is reduced.

28. A method of treatment, prevention or reduction of obesity or overweight condition-related comorbidities comprising administering a myostatin or activin antagonist or activin receptor antagonist.

29. A method according to embodiment 28 wherein obesity or overweight condition-related comorbidities are selected from the group of type 2 diabetes, glucose intolerance, prediabetes, insulin resistance, high triglycerides, physical impairment, osteoporosis , renal disease, obstructive sleep apnea, sexual hormones impairment, endocrine reproductive disorders such as polycystic ovary syndrome or male hypogonadism, osteoarthritis, gastrointestinal cancers, dyslipidaemia, hypertension, heart failure, coronary heart disease, stroke, gallstones, altered gonadal hormone profile.

30. A method of treatment, prevention or reduction of type II diabetes according to embodiments 22-29

31. A method of improving glycemic control in a patient suffering from type II diabetes comprising administering a myostatin or activin antagonist or activin receptor antagonist.

32. A method according to embodiment 31 wherein improving glycemic control is achieved by improving insulin sensitivity.

33. A method according to embodiments 30-32 wherein the patient is suffering from obesity or overweight condition.

34. A method according to any of embodiment 28-33 wherein DTSQ score (Diabetes treatment satisfaction questionnaire score), or IWQOL/IWQOL-lite (Impact of Weight on Quality of Life) score or other PRO related to obesity and/or diabetes are improved.

35. A method according to any preceding embodiment wherein the myostatin or activin receptor antagonist is an anti-ActRII receptor antibody.

36. A method according to embodiment 36 wherein the anti-ActRII receptor antibody is bimagrumab.

37. A method according to embodiment 37, wherein bimagrumab is to be administered at a dose of 3 mg/kg or 10 mg/kg.

38. A method according to embodiment 37-38, wherein bimagrumab is to be administered every 4 weeks.

Alternatively according to embodiment 39, bimagrumab is administered at a dose of 210 or 700 mg, preferably every 4 weeks.

According to any of the above aspect, wherein the type II diabetes patient is treated with background antidiabetic therapy is any approved antidiabetic medication, either in monotherapy or in combination, insulin inclusive.

Every embodiment, method or use can be combined with each other within the scope of the present disclosure.

Muscle regulation and the ActRII Receptors

Several members of the transforming growth factor beta (TGF-$\beta$) superfamily, including myostatin, activin A, and growth differentiation factor 11 (GDF11), negatively regulate skeletal muscle mass in animals and humans throughout the lifecycle. Ligand signaling occurs via type II activin receptors (both ActRIIA and B; and primarily the Smad 2/3 pathway), to inhibit muscle protein synthesis and myocyte differentiation and proliferation. The absence of any of these ligands in developing animals and humans results in a hypermuscular phenotype with an increased number and size of muscle fibers. A postpartum reduction of myostatin levels results in the hypertrophy of skeletal muscle due to an increase in the size of existing myofibers (Lee et al 2005; Lee et al 2010; Trendelenburg et al 2012). Thus, the capacity for modulating muscle growth by perturbing this signaling pathway at the receptor level is much more substantial than previously appreciated by direct anti-myostatin approaches.

Bimagrumab

Bimagrumab, the pharmaceutically active compound used in accordance with the present invention, is a fully human, monoclonal antibody (modified IgG1, 234-235-Ala-Ala, $\lambda$2) developed to bind competitively to activin receptor type II (ActRII) with greater affinity than its natural ligands that limit muscle mass growth, including myostatin and activin. Bimagrumab is cross-reactive with human and mouse ActRIIA and ActRIIB and effective on human, cynomolgus, mouse and rat skeletal muscle cells. Bimagrumab binds with extremely high affinity (KD 1.7$\pm$0.3 pM) to human ActRIIB and with relatively lower affinity to human ActRIIA (KD 434±25 pM), and is formulated for intravenous (i.v.) administration.

The manufacture of bimagrumab has been described in WO2010/125003.

Bimagrumab comprises an antigen binding site comprising at least one immunoglobulin heavy chain variable domain ($V_H$) which comprises in sequence hypervariable regions CDR1 of SEQ ID N°1, CDR2 of SEQ ID N°2 and CDR3 of SEQ ID N°3.

The use of antibodies having 1, 2 or 3 residues changed from any of the sequences of CDR1, CDR2 and/or CDR3 of the heavy chain is also comprised within the scope of the invention.

Bimagrumab also comprises antigen binding site comprising at least one immunoglobulin light chain variable domain ($V_L$) which comprises in sequence hypervariable regions CDR1 of SEQ ID N°4, CDR2 of SEQ ID N°5 and CDR3 of SEQ ID N°6 or CDR equivalents thereof. The use of antibodies having 1, 2 or 3 residues changed from any of the sequences of CDR1, CDR2 and/or CDR3 of the light chain is also comprised within the scope of the invention.

Bimagrumab also comprises a light chain of SEQ ID N°7 or SEQ ID N°8 and a heavy chain of SEQ ID N°9.

According to the invention the use of antibodies having 95% identity with the light chain and/ or the heavy chain are also comprised.

Sequence listing for bimagrumab is provided herein.

**Study Design.**

Study CBYM338X2206 was an exploratory phase Ib, randomized, double-blinded, placebo-controlled, single-center, single-dose study, conducted from 21-Aug-2012 to 25-Oct-2014 at the Profil Institute for Clinical Research, Inc., Chula Vista, CA. Sixteen healthy volunteers with insulin resistance were randomly assigned in a ratio of 10:6 to receive either a single dose i.v. infusion of 30 mg/kg bimagrumab or placebo over approximately 2 hours, followed by a 4-hour observation period. This single-dose treatment period was followed by a 24-week follow-up. All subjects were instructed to maintain their current (as of screening) level of physical activity and exercise behaviors throughout the study period. Primary endpoint was to determine the effect of bimagrumab on whole body insulin sensitivity and body composition. Patients were assessed at baseline and at 10 weeks (Day 71) after drug treatment and then followed up for 24 weeks. This clinical study was designed in accordance with the ICH Harmonized Tripartite Guidelines for Good Clinical Practice, with applicable local regulations, and with the ethical principles laid down in the Declaration of Helsinki. The study protocol and all amendments were reviewed by the independent Institutional Review Board (IRB) for the site of Sunrise, FL, 33323, USA.

**Population.** Adult male and female subjects of 18 to 65 years and who provided informed consent were included in the study. Other inclusion criteria were body mass index (BMI) of 18 to 45 $kg/m^2$ and a diagnosis of glucose intolerance, defined as blood glucose of 140-199 mg/dl at 2 hours after 75 g oral glucose administration (OGTT) or a homeostatic model assessment-insulin resistance (HOMA-IR) $\geq 2.6$. The calculation is as follows: HOMA-IR = fasting glucose (in mg/dL) $\times$ fasting insulin (in mU/L) / 405. Key exclusion criteria were any disease, in particular diabetes, patients in testosterone, growth hormone, systemic glucocorticoids therapies and women of child-bearing potential.

**Hyperinsulinemic Euglycemic Clamp.** (Le et al, 2009) After receiving a standardized evening meal (10 kcal/kg; 50% carbohydrate, 30% fat, 20% protein), followed by an overnight fast of at least 12 hours, subjects were connected to a Biostator (Life Science Instruments, Elkhart, IN, USA). One hand was placed under a heating pad to allow sampling of arterialized venous blood. Insulin was infused intravenously for 180 minutes at a rate of 1 mU.kg-1.min-1 (Step 1) to maintain arterialized venous blood glucose at the target level of 90 mg/dL. After 180 minutes, the insulin infusion rate was increased to 2.5 mU.kg-1 min-1 for a further 180 minutes (Step 2), a steady-state hyperinsulinemia was sufficient to prevent hepatic glucose output. At the end of Step 2, the insulin infusion terminated. The infusion rate of 20% v/v glucose (Glucose Infusion Rate, or GIR) in mg.kg-1.min-1 required to keep blood glucose levels at 90 mg/dL was recorded throughout the euglycemic hyperinsulinemic clamp period (Steps 1 and 2), and especially during the last 30 minutes of both periods (at quasi steady-state). The GIR was used for the calculation of insulin sensitivity indices (i.e., *M, M1,* S1). The steady-state periods for insulin sensitivity measurements were defined as the time from 150 - 180 minutes in Steps 1 and 2. The total duration of the two-step H-E clamp procedures was approximately 7 hours. Blood samples for measurement of serum insulin and glucose concentrations were drawn at 0, 150, 160, 170, 180 min in Step 1 and 150, 160, 170, and 180 min following increase to the higher insulin infusion rate Step 2.

**IVGTT.** Following a minimum 8-hour overnight fast, subjects underwent an intravenous glucose tolerance test for assessment of first phase insulin release. Basal blood samples for glucose, and insulin were drawn at -5 and 0 minutes. At 0 minutes, a bolus of glucose 0.3 g/kg of body weight was administered as D50 (50% solution in sterile water). The delivery of the bolus was completed within 2 minutes. Blood samples were drawn at 2, 4, 6, 8, 10, 15, and 20 minutes, after which the cannula was removed.

**PET FDG-18/ CT scan for BAT assessment.** Positron emission tomography (PET) with radioactive [18]fluoro-desoxy-glucose (FDG) and associated with Computed Tomography (CT) were performed during "cold" exposure and during "warm" (or thermoneutral) conditions to all subjects at baseline and week 10 (Virtanen KA et al, 2009). BAT activation under cold exposure was achieved by cooling subjects with the subject, while wearing light clothing, spending 1 hour in a room adjacent to the scanner room, set at an ambient temperature of 17-18°C. During the PET/CT scan itself, cold exposure was maintained with the scanner room temperature set to 17-18°C. The scan obtained in warm conditions (temperature at 22-24°C) was performed either the day before or the day after the one obtained under cold conditions. All scans were obtained under overnight fasting conditions. Immediately following the shoulder area dynamic acquisition, the lower leg was scanned to image the gastrocnemius and soleus muscles as examples of fast and slow twitch muscle tissue. PET Images were quantified using the time-activity curves from each voxel and from the aorta using PMOD software giving an imaging of the body metabolic rate measurements in micromoles glucose/100g/min, within a 128 x 128 x 63 matrix, with expected values in the range of 5-15. By comparing PET scans obtained with "cold" and "warm" conditions, quantitative measures were extracted from each data set.

**DXA Scan.** (Albanese et al, 2003) All subjects had a whole body dual energy X-ray absorptiometry (DXA) scan, completed at baseline, week 6, week 10, week 14 and week 24 at a centralized scan analysis (Novartis CRO). All scans required the subjects to lie supine on the DXA table and a calibration phantom was used to ensure consistency in DXA readings. From these scans, absolute values and relative changes from baseline in lean body mass (LBM) and fat-body mass (FBM) were calculated. The exam took 1-2 min. The effective dose of a DXA whole body scan was 2.1 $\mu$Sv.

**Statistical Analysis.** Analysis of the data was performed under the direction of Novartis personnel. The pharmacodynamic (PD) variable (M/I) evaluated from the hyperinsulinemic euglycemic clamp was analyzed, endpoints of interest were change from baseline values, and these endpoints were analyzed separately. Log transformation was applied to each endpoint before analysis. The log-transformed ratio to baseline was analyzed by an ANCOVA model with treatment (bimagrumab and placebo) as fixed effects and log baseline value as covariate. The treatment estimates, contrasts of treatment difference (bimagrumab-placebo) and corresponding 90% confidence intervals were back transformed. Percent relative to baseline and placebo adjusted percent relative to baseline for treatment comparison along with corresponding 90% confidence interval were provided. Descriptive statistics of raw values, percent changes and absolute changes from baseline for the PD variable were provided by time point, treatment group and step. The AUC (area under curve) and Cmax (maximum value) were derived for glucose and insulin profiles, by IVGTT. DXA parameters and body weight data as long as $HbA_{1c}$, fasting insulin and glucose were analyzed separately using mixed effects model for repeated measurements. Endpoints of interest were all change from baseline values. The log-transformed ratio for DXA parameters and body weight, and the change from baseline were analyzed by an ANCOVA model with treatment (bimagrumab and placebo), visit and the interaction visit*treatment as fixed factors, log-baseline/baseline value as covariate and subject as random factors. The treatment estimates, contrasts of treatment difference (bimagrumab-placebo) and corresponding 90% confidence intervals were back transformed. Percent relative to baseline and placebo adjusted percent relative to baseline for treatment comparison along with corresponding 90% confidence interval were provided.

## RESULTS

**Trial Population.** The healthy volunteers that were enrolled in this study were predominantly Caucasian (94%), male (81%) and largely overweight or obese (mean BMI 29.3 kg/m$^2$, range: 21.1-37.7, median 28.6 kg/m$^2$). All subjects were insulin resistant as defined by a HOMA-IR $\geq$ 2.6 and were not diabetic; mean $HbA_{1c}$ were 5.49% ($\pm$0.39) and 5.35% ($\pm$0.24) in the bimagrumab and placebo group respectively. A total of 16 subjects were enrolled, 10 in the bimagrumab group and 6 in the placebo group. Both groups were comparable with respect to baseline characteristics with the exception of subjects in the placebo group being somewhat older with a mean age 47.3 years versus 42.2 year in the bimagrumab group. All baseline laboratory findings were comparable.

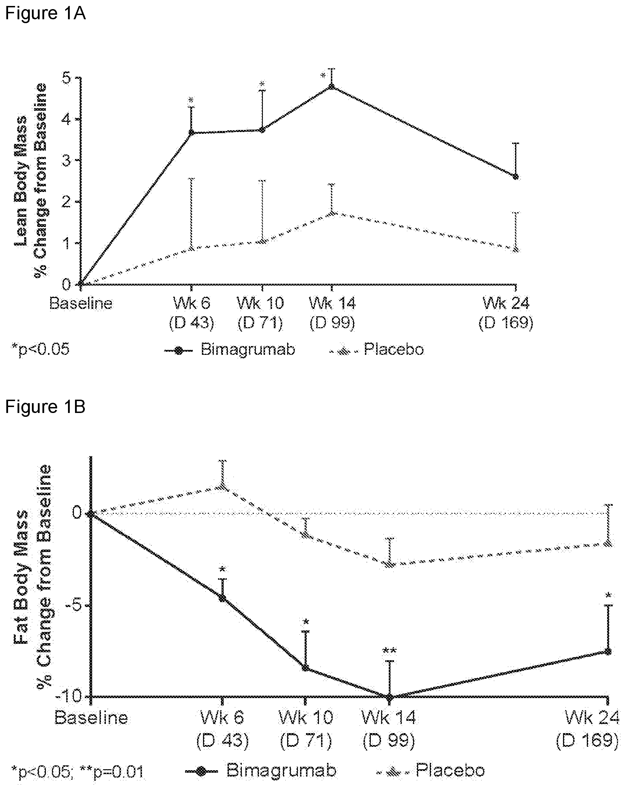

**Body composition changes.** A significant treatment effect with bimagrumab was observed on body composition at Week 10 as measured by dual energy X-ray absorptiometry (DXA) scan. As expected, the total lean body mass (LBM) increased with bimagrumab (mean of 2.7% at Week 10 [90% confidential interval (CI) 0.45, 5.01; p=0.049]) (Figure 1A), with a treatment effect that was observed as early as Week 6 (first post-dose scan) and was trending towards baseline by the end of the study (Week 24). Additionally, a significant reduction in total fat body mass (FBM) was observed at Week 10 in the bimagrumab group when compared to placebo, mean -7.9% [90% CI -12.5, -3; p=0.011]; this effect was observed as early as Week 6 (first post-dose scan) and persisted until the end of the study, mean of - 6.5% [90% CI -11.2, -1.5; p=0.034] at Week 24 (Figure 1B). Interestingly the effect of bimagrumab on LBM reached a plateau level after 6 weeks, with only an additional ~1% increase between Week-6 and Week-14, compared to the persistent decrease that was observed in FBM. There was no appreciable effect on body weight in either group throughout the study; the absolute increase of LBM with bimagrumab was mean 2 Kg ($\pm$1.4kg) compared to 750 g ($\pm$2.1 kg) in the placebo group at Week 10 compared to baseline, while the loss of absolute FBM was mean 2.3 Kg ($\pm$1.7 kg) with bimagrumab and 470 g ($\pm$0.7 kg) with placebo.

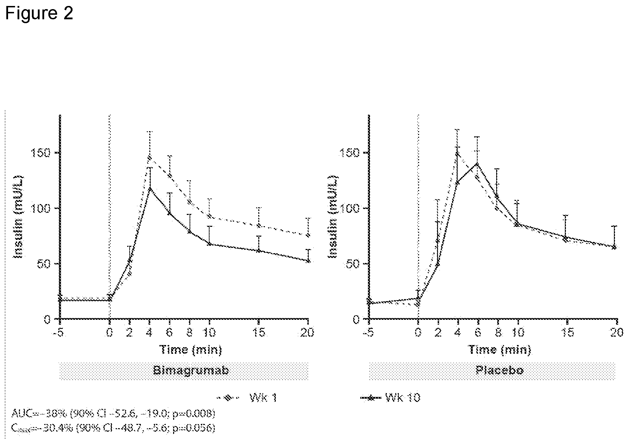

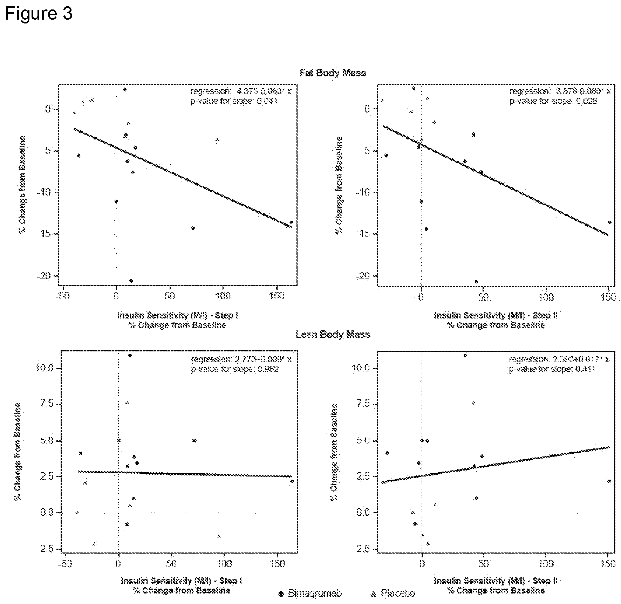

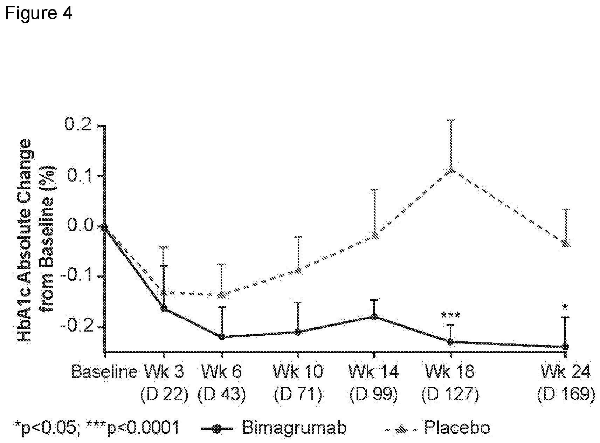

**Insulin sensitivity and $HbA_{1c}$.** Insulin sensitivity was measured by the 2 step hyperinsulinemic euglycemic clamp, and reported as the ratio of insulin sensitivity (*M*) adjusted for serum levels of insulin (*I*). The treatment effect of bimagrumab on insulin sensitivity (*M/I*) at Week 10 showed an increase by 21.8% [90% CI -8.9, 62.7; p=0.250] for Step 1 and 18.2% [90% CI -7.4, 50.9; p=0.247] for Step 2. Insulin sensitivity was also evaluated with the intravenous glucose tolerance test (IVGTT). The treatment effect of bimagrumab at Week 10 indicated a significant reduction in the insulin response to the glucose challenge with no treatment effect on glucose levels. The area under curve (AUC) for insulin was reduced by 38% [90% CI -52.6, -19; p=0.008] and the maximum concentration (Cmax) of insulin was reduced by 30.4% [90% CI -48.7, -5.6; p=0.056] (Figure 2). No differences were observed in fasting plasma glucose (p=0.674) in bimagrumab group compared to placebo group, while it was observed a significant reduction in the glucose AUC (10.3%, p=0.011) in the placebo group compared to bimagrumab group. A linear and significant relationship between loss of body fat mass and increase in insulin sensitivity at the hyperinsulinemic euglycemic clamp was observed (p=0.041 for Step 1, p=0.028 for Step 2), while no such association was observed between changes in lean body mass and insulin sensitivity (p=0.982 for Step 1, p=0.411 for Step 2) (Figure 3).The treatment effect of bimagrumab on HbA$_{1c}$ was seen as early as Week 6 and showed a reduction by 0.22% [90%CI -0.31, -0.12; p<0.001] that was sustained till the end of the follow-up at Week 24, mean -0.24% [90%CI -0.33, -0.14; p<0.001] (Figure 4). The placebo group showed a slow deterioration of HbA$_{1c}$ around Week 18, mean 0.11% [CI90% -0.01, 0.24; p=0.144], however not statistically significant.

**Brown adipose tissue (BAT).** The thermogenic capacity of BAT is defined by the difference in glucose metabolic rate (ΔGMR) measured under neutral ambient temperature (i.e. "warm" 22-24°C) and at the end of a 90-min cold exposure (i.e. "cold" 17-18°C). Prior to treatment with bimagrumab, this cold challenge resulted in a GMR increase from 0.56±0.15 μmol/100g/min to 0.67±0.24 μmol/100g/min (+19%, N=15, p=0.12). At Week 10 after drug administration, the placebo group showed a trend through a decrease in the thermogenic response; such progression into further impairment of BAT activity was not observed in the bimagrumab group (ΔGMR$_{[cold-warm]}$ placebo -0.23±0.27 μmol/min vs BYM338: 0. 06 ±0.35 μmol/min, p=0.11). A trend for a reduction in BAT volume was also observed in the bimagrumab group at Week 10 while no effect on BAT volume was detected in the placebo group (BYM338: -36.9±44.8 mL vs placebo: -4.7±18.8 mL, p=0.12).

**Muscle strength.** The treatment effect of bimagrumab on muscle strength as measured by leg press showed a trend towards an increase by 33% [90% CI -5.5, 87.1; p=0.168] at Week 14 and by 45.3% [90% CI 3.3, 104.4; p=0.073] at the end of the study (Week 24). These effects did not reach statistical significance.

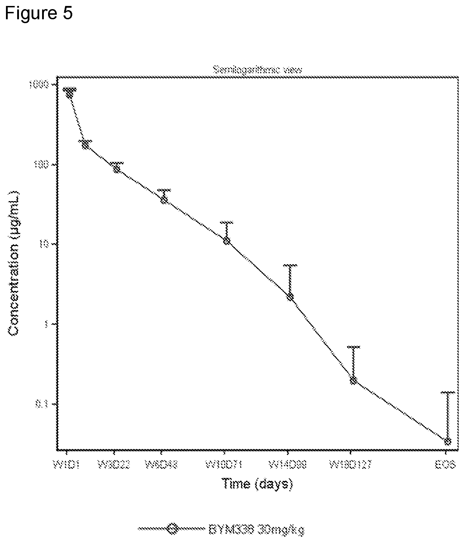

**Pharmacokinetics.** The pharmacokinetics of bimagrumab in overweight and obese individuals is consistent with that observed in lean and underweight people and exhibited a target-mediated drug disposition (TMDD) profile with a threshold for loss of the nonlinear clearance to be approximately at 10 μg/mL (Figure 5). As the loss of the nonlinear clearance could be linked to a loss of saturation of the receptors, a single dose of 30 mg/kg seemed to allow receptor saturation up to Week 10 (71 days), a time where treatment effects on body composition and insulin sensitivity were performed.

**Adverse events.** During this study, there were no serious adverse events (SAEs) and no adverse events (AEs) that led to study discontinuation. Acne, muscle spasms and myalgia were reported in 30% of bimagrumab-treated subjects and in none of the placebo-treated subjects. Diarrhea, muscle weakness and musculoskeletal stiffness were noted in 10% of the bimagrumab group and in none of the placebo group. Although upper respiratory tract infections were one of the most commonly reported AEs, they were not suspected to be related to study treatment. Anti-drug antibodies (ADAs) were detected in 2 out of 10 subjects following exposure to bimagrumab and were not neutralizing to either ActRIIA or ActRIIB. These ADAs were not associated with untoward safety findings or altered drug exposure.

**DISCUSSION**

A single dose of bimagrumab led to a reduction in HbA$_{1c}$ and in insulin sensitivity of a magnitude that is considered clinically meaningful in insulin resistant, non-diabetic individuals. These metabolic effects of bimagrumab were associated with profound changes in body composition, in the absence of a meaningful decrease in body weight or change in lifestyle. The Look AHEAD trial demonstrated that weight loss of 5-10% of body weight at one year induces clinically significant improvements in cardiovascular risk factors and The Diabetes Prevention Program showed that 5% weight loss reduced the 5-year risk of developing new type 2 diabetes by 58% (Diabetes Prevention Program Research Group, 2002). The risk reduction can be linked to decreases in visceral fat mass in response to these combined interventions. A single dose of bimagrumab led to a significant increase in total LBM of 2.7% (2Kg ±1.4) while the addition of exercise to a weight loss regimen with normal protein intake (1.2 g/kg/day) at best maintains baseline total LBM, and has not been shown to increase lean mass (Longland et al., 2016). Moreover bimagrumab led to a profound and lasting reduction in total FBM of 7.9% (2.3 Kg ±1.7) at Week 10, a combined effect which, to our knowledge, was never observed with any other anti-obesity drug up to now. The net result of these changes is a neutral body weight effect combined with markedly reduced body adiposity (i.e. reduction of the percentage of fat mass in soft tissue mass), which resonates with a healthier and fitter body composition. The metabolic impact of these body composition changes on insulin sensitivity and thereby glycemic parameters was significant even in a heterogenous group of insulin-resistant subjects. Thus, bimagrumab has a novel mechanism of action to tackle the metabolic alterations of adiposity. Further studies are needed in patients with obesity, particularly those with central obesity/adiposity, to further explore whether sustained increases in lean body mass by longer treatment with bimagrumab can also promote body weight loss with continuing relative reduction in tissue adiposity, especially if combined with a calorie-restricted diet.

The improvement in $HbA_{1c}$ probably reflects a decrease in post-prandial glucose levels, as no effect was observed on fasting glucose levels. The effect on postprandial glucose level may reflect an increase in peripheral insulin sensitivity. The observed translation of the treatment effect on decreased $HbA_{1c}$ is higher than that obtained with metformin or lifestyle intervention ($HbA_{1c}$ down ~ 0.1% at 5 months) (Diabetes Prevention program Research Group, 2002) while it is comparable with that observed with liraglutide 3.0 mg qd in a similar population ($HbA_{1c}$ down 0.23% at Week 56) (Pi-Sunyer et al, 2015). The effect of bimagrumab on insulin sensitivity as measured by the hyperinsulinemic euglycemic clamp and by the IVGTT, is on par with the effects of pioglitazone (45 mg) and liraglutide (1.8 mg) along with caloric restriction in a similar population, although the indices used slightly differ between the three studies (DeFronzo et al., 2011; Kim et al, 2013; Matsuda et al, 1999). Further and larger studies are needed to explore whether extended treatment with bimagrumab exerts larger effects when administered to a more homogenous group of insulin-resistant type 2 diabetics.

Overall bimagrumab was safe and well tolerated with no major safety findings in this new population of mainly overweight and obese individuals. Adverse events were minor and transient, as well as consistent with the previous clinical experience with bimagrumab, namely acne, particularly at dose levels used in this study, isponatenous muscle contractions and myalgia. The biological explanation for acne remains unclear and isbe explored further. The key findings from this study of short treatment duration included the combination of rapid increases in lean body mass and decreases of fat mass, which collectively confers profound decreases in body adiposity without changes in body weight. These changes shifted body composition towards a "fit-fat" phenotype without utilization of physical exercise and dietary restrictions.

Furthermore, these shifts in body composition in the absence of weight loss mirrored the metabolic impact of weight loss with visceral involvement on glycemic parameters.

Limitations of this trial include the fact that this is a pilot exploratory study with a small number of subjects; the population was heterogeneous for body mass index (the trial included normal weight, overweight and obese individuals) and body fat distribution. Hence, the evaluation of the effect size of bimagrumab on insulin sensitivity and $HbA_{1c}$ was limited. This study did not and could not evaluate the effect of bimagrumab on the various compartments of body fa, thus we cannot comment on effects on visceral, subcutaneous and liver fat content. The mechanism for the observed improvement in insulin sensitivity was not systematically investigated; it could be secondary to reduced intra myocellular fat (Kuhlmann et al, 2003) content, and/or to overall reduction in fat mass since it was observed a significant relation between FBM and insulin sensitivity (*M/I*) measured with the clamp, which was not observed with LBM (Figure 3). In addition, it could also involve increases in circulating adiponectin, which was observed in earlier studies testing repeated dosing with bimagrumab in healthy volunteers.

Recent studies with bimagrumab 10 mg/kg treatment given over at least 6 months demonstrated that while the effect on skeletal muscle mass (hypertrophy) tends to reach maximum after 2-3 months of treatment, the effect on body fat mass is increasing with longer treatment and ultimately exceeds the magnitude of the muscle gain, thereby promoting weight loss. In addition, regional measurements with DEXA indicated that the predominant part of body fat mass loss is coming from the trunk, which encompasses both subcutaneous and visceral fat mass. Although, conventional DEXA cannot differentiate these two components this observation does not exclude the possibility of potential involvement of the visceral fat depot. Further and larger studies utilizing more advanced imaging techniques (such as whole-body CT or MRI) are needed to clarify whether these effects on truncal fat mass during extended treatment with bimagrumab can confer particular benefits in patients with central adiposity (typically insulin-resistant and type 2 diabetic patients) and if so how this translates to improvement in insulin sensitivity, metabolic profile and ultimately clinically meaningful reductions in $HbA_{1c}$.

The contribution of increases in hepatic insulin sensitivity to the observed beneficial effects of bimagrumab is not known. However, previous studies on bimagrumab - in line with preclinical observations on anti-myostatin antibodies - have indicated dose-dependent increases in circulating adiponectin levels. Adiponectin is a fat-derived hormone with known direct actions in the liver that can confer improved hepatic insulin sensitivity, in addition to conferring insulin sensitivity in skeletal muscle. We are speculating that decreased tissue adiposity in skeletal muscle (by increase in lean mass combined with decreases in intermuscular fat mass) and decreases in systemic and local inflammation could result in improved muscle contractility and thereby function performance and mobility in these individuals. The effect on muscle strength by 1-leg press is suggestive of a trend, but findings were not conclusive due to the small sample size and the heterogeneity of participants. Further studies with larger sample size are warranted to explore this question in a more targeted manner. In conclusion, bimagrumab may offer a novel approach for the treatment of metabolic complications of obesity such as insulin resistance. It is also possible that bimagrumab could be a novel insulin sensitizing therapeutic for type 2 diabetes through a meaningful effect on body composition and increases in adiponectin. Unlike available therapeutics for type 2 diabetes, which tends to increase weight and fat mass, bimagrumab could reverse important features of the underlying pathophysiology of central adiposity and thereby type 2 diabetes.

## Example 2 : Investigational plan

### Study design

BYM338X2211 is a non-confirmatory, randomized, subject and investigator blinded, placebo-controlled, parallel arms study, investigating a 48-week treatment period with intra venous bimagrumab in overweight/obese patients with type 2 diabetes. Approximately 60 patients are enrolled and randomized. For patients who do consent for the optional MRI, their liver, visceral and subcutaneous fat content are assessed.

| Population | Approximately 60 obese patients (BMI:28-40 inclusive) with Type 2 Diabetes<br>Males and Females between the ages of 18-65 inclusive |
|---|---|
| Key Inclusion criteria | • Male and female, age 18 to 65 years (inclusive), in stable health condition as determined by past medical history, physical examination, vital signs, electrocardiogram, and laboratory tests at screening.<br>• Type 2 diabetes, with an HbA1c between 7% and 10% (inclusive) at screening, on metformin or DPP4 inhibitor agent monotherapy, with stable treatment for approximately 3 months prior to randomization.<br>• Body mass index (BMI) of 28 to 40 kg/m$^2$ (inclusive) at screening.<br>• Body weight between 65 and 140 kg (inclusive) at screening, and with a stable body weight ($\pm$5 kg) by history (patient report) and stable physical activity within 3 months prior to screening.<br>• At screening vital signs should be as follows: oral body temperature between 35.0-37.5 °C<br>• systolic blood pressure, 90 to 150 mm Hg<br>diastolic blood pressure, 50 to 90 mm Hg<br>pulse rate, 50 - 100 bpm |
| Key Exclusion criteria | • Pregnant or nursing (lactating) women, where pregnancy is defined as the state of a female after conception and until the termination of gestation confirmed by a positive hCG laboratory test.<br>• Women of child-bearing potential, defined as all women physiologically capable of becoming pregnant, unless they are using highly effective methods of contraception during dosing and for 6 months after stopping of investigational drug.<br>• Diabetes other than Type 2 such as Type 1 diabetes, surgically induced-diabetes; "brittle" type 2 diabetes as per investigator judgment, history of severe hypoglycemic epidoses in the year preceding screening, or hypoglycemic unawareness.<br>• History of clinically significant arrhythmias, unstable angina, myocardial infarction or stroke, coronary artery bypass graft surgery, or percutaneous coronary intervention (e.g. angioplasty or stent placement), within 6 months of screening or 1 year for drug-eluting stents.<br>• Use of any anti-obesity medications, nutritional supplements or over the counter products for weight loss within 3 months of screening. Use of medications known to induce weight gain such as some anti convulsant and psychotropic medications (e.g. clozapine) within 3 months of screening. |

### Screening (Days -21 to -8)

Participants undergo an onsite screening visit to determine their eligibility for the study Subjects who qualify for enrollment following screening are scheduled for baseline assessments.

Planned lifestyle interventions (U.S. Department of Health and Human Services Food and Drug Administration Center for Drug Evaluation and Research 2008) include dietary counseling for weight loss with a daily caloric deficit of 500 kcal, with a diet that follows the American Diebetes Association (ADA) guidance for optimal glycemic control, and with protein intake of at least 1.2 g/kg/day to support muscle anabolism. Patients receive counseling for physical activity and are encouraged to follow the U.S Department of Health and Human Services (2008) guidelines (please refer to the SOM). These interventions are initiated at screening once eligibility is confirmed.

### Baseline (Days -7 to -1)

Prior to dosing (Day 1), patients who are eligible for enrollment following screening return to the clinic to undergo baseline assessments. To facilitate study conduct, patients may opt to be domiciled at Day -1 to complete baseline assessments prior to dosing on Day 1.

**Randomization and Dosing (Day 1)**

Eligible patients, based on screening and baseline assessments are randomized in a 1:1 ratio to receive either bimagrumab or placebo. Randomization : patients are stratified according to baseline BMI into 2 strata:

- BMI between 28 kg/m$^2$ and 33kg/m$^2$ (inclusive) and
- BMI above 33 kg/m$^2$ and up to 40 kg/m$^2$ (inclusive).

Administration of bimagrumab or placebo is done via an intravenous infusion over 30 minutes followed by an observation period that includes safety and tolerability and PK sampling. Following all assessments, patients may be discharged from the Investigator site when the Investigator judges them to be medically stable, in good general health and not needing further observation.

**Treatment period (Days 1 - 336)**

Patients continue on background monotherapy as per eligibility criteria (see Inclusion criteria) throughout the study. All participants can continue to receive care for their diabetes and adaptation of their background therapy can be made.

Administration of bimagrumab or placebo is done via an intravenous infusion over 30 minutes followed by 1-hour observation period once every 4 weeks for a total of twelve doses. Bimagrumab is dosed based on body weight at 10 mg/kg, with a dose cap of 1200 mg for body weight equal to and above 120 kg. Placebo is provided as D5W, 5% Dextrose solution.

Patients receive regular monitoring and advice on diet and physical activity as part of their monthly site visits throughout the study.

Patients are asked to return to the Investigator site for dosing approximately every 4 weeks during the treatment period. During these visits, patients are evaluated for safety, tolerability, PK and efficacy.

The treatment period ends 4 weeks after the last administration (on Day 308/Week 44).

**Follow Up (Days 364 -392)**

After completion of the treatment period patients have a follow-up period of 8 weeks with regular monitoring for safety and efficacy (at Week 52) until the end of study visit (EOS) which take place 12 weeks after the last study drug administration .

**Rationale of study design**

The rationale for key elements of the study design include:

- **Randomization:** To decrease the chance of an imbalance in subject characteristics (e.g. age, BMI) between treatment groups.

- **Stratification:** BMI was selected as a stratification parameter as it is an important predictor of response on parameters of body composition/body weight and HbA1c (internal data). The expected median value for BMI in this patient population is 33 kg/m$^2$ (internal data), therefore 2 strata above and below/inclusive of the median ensure a balanced representation of subjects between placebo and active in each strata, i.e. strata of approximately similar size between the 2 arms.

- **Subject- and investigator-blinding:** To mitigate the risk of bias in treatment allocation, reporting and causality assessment of adverse events. Furthermore, this design decreases the potential confounding effect of intentional or unintentional behavioral changes made by subjects that are aware of their treatment assignment

- **Placebo arm:** The inclusion of the placebo provides a comparison group with bimagrumab and is included to allow for the double-blind design.

- **Lifestyle interventions:** Trials with anti-obesity agents have to demonstrate treatment benefits on body weight/-composition on a background of first line-therapy with lifestyle interventions. The daily caloric deficit of 500 Kcal is a standard approach and is expected to induce weight loss over the treatment period. It may also enhance the effect of bimagrumab on body composition and body weight. The American Diabetes Association (ADA) walking program is tailored to the type of population in this study and is a gentle, easy to implement approach for physical activity. Exercise is known to enhance the effect of bimagrumab on muscle function, which may support the treatment benefit of bimagrumab on body composition and weight.

- **Adequate protein intake:** this is important for optimal muscle maintenance/growth, the protein content of 1.2 g/kg/day is recommended to compensate for the caloric deficit.

**Standard of care diabetes therapy:** Patients remain on their standard of care treatment for diabetes, enabling the evaluation of added treatment benefit with bimagrumab on glycemic parameters. Oral diabetes monotherapy supports selection of patients who are early in their disease state and thus without significant co-morbidities. Monotherapy is restricted to Metformin or DPP4 inhibitors, as these medications are less likely to affect body weight and thus confound the study results

**Rationale for dose/regimen, route of administration and duration of treatment**

**Dose rationale**

In healthy volunteers (HV) and sIBM patients, a 10 mg/kg dose of bimagrumab was shown to provide exposure levels (i.e. above 10 $\mu$g/mL) at which the anabolic effect is observed and maintained over dosing intervals of 4 weeks [CBYM338X2102 (N=6 subjects), CBYM338X2104 (N=47 subjects)], for up to six doses [CBYM338X2109 (N=35 subjects)] and up to one year [CBYM338B2203 (N=54 sIBM patients)]. The threshold for minimal target exposure for bimagrumab is approximately 10 $\mu$g/mL, a concentration below which nonlinear clearance is observed, suggesting loss of full receptor saturation and target-mediated drug disposition. In clinical studies to date, bimagrumab concentrations approximately at or above 10 $\mu$g/mL for at least 4 weeks in HV and more than one year in sIBM patients has been safe, well tolerated, demonstrated an increase in thigh muscle volume. The 26-week toxicology studies in Cynomolgus monkeys showed a chronic exposure at NOAEL (300 mg/kg/week) of approximately 300-fold and 55-fold for AUC and Cmax respectively when compared to human exposures at 10 mg/kg at steady state.

Dosing in this study is weight based for patients with body weight up to 120 kg, and is capped at 1200 mg for patients with body weight between 120 kg and 140 kg. Body-weight based dosing has proven to reduce variability in exposure in subjects/patients, and is implemented as applicable. Capped dose is selected for body weights > 120 kg because of the uncertainty of the effect of large body weight and body composition (% fat mass vs. % lean mass) on the pharmacokinetics, exposure, and safety profile of bimagrumab. To date, the pharmacokinetic data is limited in obese subjects, and the maximal body weight in dosed subjects has been 116 kg, in studies conducted with bimagrumab in overweight to obese subjects with insulin resistance (N=10), and obese healthy subjects (N=6). The maximal amount of bimagrumab administered to-date is 3500 mg (at a dose of 30 mg/kg), given i.v. and as a single dose for a maximal body weight of 116 kg. This dose did not show over-exposure and caused no safety concerns. A capped dose for these subjects is selected to avoid over-exposure and to maintain bimagrumab levels around the threshold for safe anabolic effects over 4-week dosing intervals. Specifically, the selected amount of 1200 mg translates to a body weight based dose ranging from 10 to 8.6 mg/kg for the body weight range of 120-140 kg, which is predicted to result in exposure levels within the safe and efficacious range for bimagrumab and with minimal risk of over-exposure.

**Rationale for duration of treatment**

A treatment duration of 48 weeks is selected to capture the temporal profile as well as maximal effect of bimagrumab on body fat mass. While a ceiling effect is typically observed on lean mass gain with bimagrumab, the loss of fat mass does not seem to plateau over a period of 24 weeks and even up to 64 weeks (Internal data).

**Rationale for follow-up period**

The extended follow-up period of 8 weeks is selected to monitor the durability of treatment effect of bimagrumab on body fat mass, lean mass and glycemic control off treatment. The EOS visit being performed 12 weeks after the last administration covers the wash-out period of bimagrumab exposure associated with anabolic effect (approximately 8 weeks).

Rationale for choice of comparator

A placebo is used as a comparator in this study based on the following rationale:

- A placebo comparator is needed to maintain the double blind design.

- The placebo group is not intended as a primary efficacy comparator to bimagrumab, as the change from baseline in the bimagrumab treated group is the primary determination of efficacy.

Rationale for choice of background therapy

This is an anti-obesity trial in patients with T2D with the primary goal being the effect of bimagrumab on body composition. While improvement in glycemic control may occur as a result of improved body composition,, patients still need to maintain their background T2D therapy throughout to avoid a deterioration in glycemic control. T2D treatment is restricted to monotherapy for homogeneity of the study population and to enable interpretability of the data. Monotherapy is restricted to classes with minimal effect on body weight, including metformin, a first line therapeutic agent, and DPP4 inhibitors. If improvement in glycemic control is observed during the study, reduction in anti-diabetic treatment is allowed to prevent hypoglycemia.

**Treatment**

**Investigational treatment and control drugs**

The investigational drug, BYM338 (bimagrumab) 150mg LIVI (liquid in vial), is prepared by Novartis and supplied to the Investigator site as open labeled bulk medication. Study drug preparation is detailed in a separate pharmacy manual. Placebo is a dextrose 5% (D5W) in water infusion supplied by the site.

**Table 1 Overview of study medication**

| Study drug name | Formulation | Appearance (e.g., approximate size, color, etc.) | Packaging | Provided by |
| --- | --- | --- | --- | --- |
| BYM338 (bimagrumab) | liquid in vial | water infusion | open labeled bulk medication | Novartis |
| Placebo | liquid | water infusion | | site |

Study drugs must be received at the study site by a designated person, handled and stored safely and properly, and kept in a secure location to which only the Investigator and designated staff have access. Upon receipt, the study drugs should be stored according to the instructions specified on the drug labels. Storage conditions must be adequately monitored and appropriate temperature logs maintained as source data. Appropriate documentation of the patient specific dispensing process must be maintained. Bulk medication labels are in the local language, comply with the legal requirements of each country, and includes storage conditions for the drug but no information about the patient.

**Additional study treatment**

No additional treatment beyond investigational drug and control drug are included in this trial.

**Background therapy**

Metformin or DPP4 inhibitor are requested as background therapy for patients to be eligible in the study.

**Treatment arms**

Patients are assigned to one of the following 2 treatment arms in a ratio of 1:1
Study treatments are defined as:

- BYM338 (bimagrumab) 10 mg/kg up to maximum 1200 mg monthly (12 doses)
- Placebo monthly (12 doses)

Efficacy / Pharmacodynamics

Pharmacodynamic assessments are specified below, with the methods for assessment and recording specified in the Site Operations Manual (SOM). Assessments are performed/samples collected at defined time points.

MRI and DXA images is centrally read by a CRO.

Pharmacodynamic (PD) samples is obtained and evaluated in all patients.

1. Glucose control assessment

Fasting insulin and glucose

Fasting glucose and insulin are taken at different times.

HbA1c

HbA1c reflects average glucose concentrations over the past 3 months and therefore provides a useful index of the glycemic control of bimagrumab over that time period. It is a standard endpoint used to assess the glycemic efficacy of any anti-diabetic medication. HbA1c is a key glycemic parameter which correlates with reduction of risk of diabetic complications.

HOMA-IR

Patients undergo fasting insulin and glucose at screening to estimate the degree of insulin resistance using the homeostatic assessment model of insulin resistance (HOMA-IR) and inverse of the HOMA-IR.

QUICKI

QUICKI is being evaluated as it is a better estimate of insulin resistance than HOMA-IR in patients with diabetes and elevated fasting glucose levels, e.g. > 170 mg/dl (Yokoyama et al 2004). QUICKI is a derived value of insulin sensitivity index using fasting glucose and insulin levels and provide additional and complementary information to that obtained with HOMA-IR (Hrebicek et al 2002).

2. Imaging

DXA Scan

Dual energy X-ray absorptiometry (DXA) is used to assess changes in body composition, including total fat and lean body mass (FBM and LBM) and appendicular skeletal fat and muscle mass (aFBM and aLBM). DXA instruments use an x-ray source that generates and is split into two energies to measure bone mineral mass and soft tissue from which fat and fat-free mass (or lean body mass) are estimated. The exam is quick (~5-6 min), precise (0.5-1%) and non-invasive. DXA scanners have the precision required to detect changes in muscle mass as small as 5%.

Quality assurance is an important issue in the use of DXA scans to determine body composition. DXA instrument manufacturer and model should remain consistent and their calibration should be monitored throughout the study. Use of a standardized scan acquisition protocol and appropriate and unchanging scan acquisition and analysis software is essential to achieve consistent results. Likewise, because of variability in interpretation of the scans, it is important to utilize centralized scan analysis by experienced staff.

Data collection and processing is explained in the imaging charter written by the imaging CRO supporting the study.

MRI scan

Magnetic resonance imaging (MRI) is used to assess changes in the percentage of fat in the liver (% fat fraction or %FF), the visceral and subcutaneous adipose tissue volumes in the abdominal region, as well as the paravertebral muscle cross-sectional area and associated fat contents (both the inter-muscle adipose tissue-IMAT and muscle FF contents). All images are acquired in the axial plane by using an imaging pulse sequences optimized for water/fat separation and adapted to the MRI system capabilities.

3. Anthropometric measurements

- Height

- Body weight

- Waist circumference

- Hip circumference

- Waist to hip ratio

- Body mass index (BMI) is calculated (Body weight (kg)/ [Height (m)]$^2$)

4. Performance measurements of physical function

Exercise Program

ADA walking guidelines are encouraged. Please refer to the SOM for additional details.

Timed Chair Stand

The timed chair stand test resembles a component of the Short Physical Performance Battery (Patel et al 2014), which assesses a person's ability to rise from a chair without the use of the arms once and then multiple times consecutively. This test requires no advanced technology and can be administered within a clinic or similar sized space. A description of the chair stand test, including the list of equipment, set up and script of instructions are available in the SOM.

Hand-Grip Strength Test

The purpose of this test is to measure the maximum isometric strength of the hand and forearm muscles. As a general rule, people with strong hands tend to be strong elsewhere, so this test is often used as a general test of strength.

5. Patient reported outcome

PRO: Impact of Weight on Quality of Life-Lite

The Impact of Weight on Quality of Life-Lite (IWQOL-Lite) is a survey instrument that is used to quantitatively assess an individual's perception of how their weight affects their day-to-day life. This instrument is especially valuable to validate the effectiveness of the treatment for obesity using metrics that go beyond the physical measurements of weight loss.

DTSQ (Diabetes Treatment Satisfaction Questionnaire)

The Diabetes Treatment Satisfaction Questionnaire (DTSQ) was first developed in the early 1980s. It is now widely used, particularly in clinical trials, but also for routine clinical monitoring. It is available in more than 100 languages. The original DTSQ is now referred to as the status version (DTSQs) in order to distinguish it from the DTSQ change version (DTSQc) which has been developed to overcome potential ceiling effects (i.e. where respondents score maximum or near-maximum satisfaction at baseline and can show little or no improvement at follow-up).

Analysis of the primary variable(s)

The primary aim of the study is to assess the effect of bimagrumab on fat mass at Week 24 and Week 48 of the treatment period.

Variable(s)

The primary efficacy variable is the change from baseline in fat mass at Week 24 and at Week 48.

Statistical model, hypothesis, and method of analysis

The study design enables evaluation of efficacy based on the following dual criteria 1) statistical significance (superior treatment effect, 1-sided 10% level) in fat mass ; and 2) clinical relevance of the change in fat mass (estimated median treatment effect of 5% or more). Weight loss of 5% has been shown to translate into clinical benefit in an overweight/obese population with T2D (Franz et al 2015). Fat mass loss of a similar magnitude to the weight loss is expected to translate into similar clinical benefits, such as on glycemic control in a similar population.

The randomization is stratified by BMI category (>=28 kg/m2 and <=33 kg/m2, >33 kg/m2 to <=40 kg/m2) in order to achieve an approximate balance of BMI distribution across the two treatment groups. The cutoff value of 33 kg/m2 represents the expected median BMI in that population (based on internal data), therefore the two randomization strata are expected to be of similar size. However, equal size strata is not enforced. A minimum of 10 patients is targeted for enrollment in the smaller stratum to ensure accurate precision on the treatment effect in both strata.

A longitudinal model describing fat mass over time is used (time modeled as a continuous variable), using all of the data collected from both randomization strata and adjusting for baseline fat mass, treatment arm, baseline BMI, with a random intercept and a random slope. The change in fat mass at Week 24 and Week 48 is estimated from that model. As a supportive analysis, the proportion of patients reaching at least 5% fat loss at Week 24 and Week 48 is presented by treatment group.

Handling of missing values/censoring/discontinuations

The primary analysis model described above is valid under the assumption of data missing at random. If the dropout rate is greater than 10% in any arm, other analysis methods is used to assess the sensitivity of the results to different methods for missing data handling.

Sensitivity analyses

Other models may be used (such as pattern-mixture models) if the dropout rate is higher than expected, in order to assess sensitivity of the primary efficacy conclusions to missing data.

Analysis of secondary variable(s)

A secondary efficacy variable of particular interest is the change in HbA1c at Week 24 and Week 48.

Other parameters of glucose control and insulin sensitivity (fasting glucose and insulin, HOMA-IR, QUICKI) and anthropometric body measurements (body weight, BMI, waist circumference, waist-to-hip ratio and lean body mass (LBM) as measured by DXA) are other secondary efficacy variables.

Efficacy / Pharmacodynamics

The secondary variable of HbA1c is analyzed in a similar fashion to fat mass, to assess the statistical significance (superior treatment effect, 1-sided 10% level) of bimagrumab therapy on HbA1c, and the clinical relevance of this effect (median treatment effect of 0.5%). A model is used to describe HbA1c over time and the change in HbA1c at all timepoints of interest (including Week 48) is estimated from that model. The analysis considers observations censored after a change in background anti-diabetic medication or dose. This analysis is expected to be unbiased because adjustments for background medication/dose are based on observed data (HbA1c, FPG), making the censored data following the medication change likely missing at random (MAR). Other adjustments for background anti-diabetic medications may be considered in the model. As a supporting analysis for metabolic changes, summaries of increase (and decrease) in background anti-diabetic medication may be done. A change in background anti-diabetic medication is defined as a change in daily dose and/or the addition of a second agent.

**DEFINITIONS**

The term "obesity" is based on BMI for both youth and adults, but the definitions are not directly comparable. Among adults, there is a set cut point based on health risk, while among children the definition is statistical and is based on a comparison to a reference population. BMI was calculated as weight in kilograms divided by height in meters squared, rounded to one decimal place. Obesity in adults is defined as a BMI of greater than or equal to 30 kg/m$^2$. Obesity in youth is defined as a BMI of greater than or equal to the age- and sex-specific 95th percentile of the 2000 CDC growth charts.

The term "overweight condition" is based on a BMI $\geq$ 25 - < 30 kg/m$^2$.

Overweight condition may also be associated with at least one additional risk factor for fatal diseases (stroke, MI, heart failure, sudden death) such as diabetes, hypertension, family history of premature coronary artery disease, etc.),

In addition overweight condition herein may be associated with at least one additional risk factor for fatal diseases (stroke, MI, heart failure, sudden death) such as diabetes, hypertension, family history of premature coronary artery disease, etc.)

Because different subjects can have same BMI but different percentage of fat and muscle mass, BMI is not always a good index to classify overweight and obesity. A high percentage of muscle mass can lead to a high BMI even with a small percentage of fat; in this case the subject may be wrongly considered overweight or obese, based on the BMI classification. Other indexes in addition to BMI are used, namely waist circumference and a body shape index - ABSI-(Bouchi et al.,2015); imaging, by DXA and MRI, is often used in clinical trials to quantify the percentage of muscle, fat and the fat distribution.

The term "body composition" is used herein to describe the percentages of fat and muscle in human bodies. Because muscular tissue takes up less space in our body than fat tissue, our body composition, as well as our weight, determines leanness.

Two people of same sex and body weight may look completely different from each other because they have a different body composition.

"Lean body mass" is a component of body composition, calculated by subtracting body fat weight from total body weight: total body weight is lean plus fat.

In equations:

$$LBM = BW - BF$$

Lean body mass equals body weight minus body fat

$$LBM + BF = BW$$

Lean body mass plus body fat equals body weight

The percentage of total body mass that is lean is usually not quoted - it would typically be 60-90%. Instead, the body fat percentage, which is the complement, is computed, and is typically 10-40%. The lean body mass (LBM) has been described as an index superior to total body weight for prescribing proper levels of medications and for assessing metabolic disorders, as body fat is less relevant for metabolism.

The term "fat mass" refers to that portion of the human body that is composed strictly of fat. It can be measured with dual energy absorptiometry DXA, MRI or bioelectrical impedance techniques.

The term "central adiposity refers to the following:

Obesity is defined as a condition of abnormal or excessive fat accumulation in adipose tissue. The amount of excess fat in absolute terms, and its regional distribution between different fat depots both play an important role in determining the health impact of obesity. Obesity can be categorized into central/android obesity and peripheral/gynoid obesity, android obesity being more typical for men while gynoid obesity being more characteristic for women.

There is substantial evidence in the literature arguing that not all obesity are associated with adverse metabolic profile and increases in cardiovascular risk. In fact, body fat distribution (i.e. relative presence of abdominal versus peripheral fat mass) was deemed as a better indicator of the metabolic and cardiovascular risks than the degree of obesity per se. In men, who tend to accumulate fat in the truncal region, increasing BMI is associated with increasing CV risk, while in women BMI is a generally poor indicator/surrogate of cardiovascular risk. Trunk fat mass can be subdivided into subcutaneous (SC) fat (in the abdominal wall) and visceral adipose tissue (in the intra-abdominal cavity). Subcutaneous and visceral fat differ significantly in terms of their anatomy, cellular composition, endocrine function and cellular regulation. VAT compared with SC is more cellular, vascular, innervated and infiltrated by inflammatory and immune cells, which translate to a higher metabolic activity and increased release of pro-inflammatory cytokines with direct and indirect implications for insulin resistance, type 2 diabetes, and the risk of cardiovascular disease. In contrast, subcutaneous fat mass, especially fat depots of the thigh and buttocks, were associated with constitutive secretion of adiponectin conferring insulin sensitizing, anti-inflammatory and anti-atherogenic effects. Low-grade inflammation has been associated with muscle wasting, which in turn may further worsen insulin sensitivity and add to the relative risk of developing type-2 diabetes. Therefore, an imbalance between central and peripheral fat depots (central adiposity) even without manifest obesity (i.e. BMI <30 kg/m2) can be linked to pronounced insulin resistance, metabolic alterations and systemic low-grade inflammation collectively driving accelerated atherogenesis.

In clinical practice, anthropometric measurements such as waist circumference or the waist-to-hip ratio are widely used to estimate abdominal obesity, but these anthropometric measures are not able to distinguish between visceral and subcutaneous fat in the abdominal region and hence are associated with inaccuracies. More advanced technologies, such as computed tomography (CT) or dual energy X-ray absorptiometry (DXA) can provide more direct assessment/measurement of fat mass. CT has an advantage in distinguishing between VAT and SAT, whereas DXA is useful for assessing the distribution of body fat mass between predefined anatomic regions (arms, legs, trunk) and help to distinguish android from gynoid adiposity.

The term "type II diabetes" referred as type 2 diabetes, previously referred to as "non-insulin-dependent diabetes" or "adult-onset diabetes," accounts for 90-95% of all diabetes, encompasses individuals who have insulin resistance and usually relative (rather than absolute) insulin deficiency. At least initially, and often throughout their lifetime, these individuals may not need insulin treatment to survive. There are various causes of type 2 diabetes.

Although the specific etiologies are not known, autoimmune destruction of B-cells does not occur, and patients do not have any of the other known causes of diabetes. Most, but not all, patients with type 2 diabetes are overweight or obese. Excess weight itself causes some degree of insulin resistance. Patients who are not obese or overweight by traditional weight citeria may have an increased percentage of body fat distributed predominantly in the abdominal region. Type 2 diabetes frequently goes undiagnosed for many years because hyperglycemia develops gradually and, at earlier stages, is often not severe enough for the patient to notice the classic diabetes symptoms. Nevertheless, even undiagnosed patients are at increased risk of developing macrovascular and microvascular complications.

The term "comorbidities of obesity or overweight" : Obesity is associated with serious chronic disorders including but not limited to type 2 diabetes or glucose intolerance, prediabetes, high triglycerides, physical impairment, osteoporosis , renal disease, obstructive sleep apnea, sexual hormones impairment, endocrine reproductive disorders such as polycystic ovary syndrome or male hypogonadism, osteoarthritis, gastrointestinal cancers, dyslipidaemia, hypertension, heart failure, coronary heart disease and stroke, gallstones, hypertension, altered gonadal hormone profile.

"Glucose intolerance" is defined as the inability to properly metabolize glucose.

"Insulin sensitivity" describes how sensitive the body is to the effects of insulin. Someone said to be insulin sensitive will require smaller amounts of insulin to lower blood glucose levels than someone who has low sensitivity. Insulin sensitivity varies from person to person and doctors can perform tests to determine how sensitive an individual is to insulin.

"Insulin resistance" is defined as a condition of tolerance to insulin, making the hormone less effective, causing decreased glucose uptake in muscle tissue that result in impaired glucose oxidation and glycogen synthesis, and a deficient suppression of hepatic glucose production in the liver. In obese, increased visceral fat mass with elevation of plasma free fatty acid (FFA) caused by the intensified lipolytic activity, worsen insulin resistance through the impairment of insulin action (Reaven et al., 1988); a mechanism known as lipotoxicity (DeFronzo, 2004).

Herein the terms "improving insulin sensitivity" and "treating/lowering insulin resistance" shall be construed as equivalent.

High concentrations of FFA in skeletal muscle cells lead to reduction in insulin-stimulated intracellular transport of glucose through the Glut4 transporter (Dresner et al., 1999), in the hepatocytes lead to enhanced rate of gluconeogenesis and glucose release from the liver and augmented insulin secretion from β cells in response to a transient increase of FFA or an inhibition effect in response to chronic elevated levels (Boden, 1997). As a result of insulin resistance and lipotoxicity, more insulin is needed to induce glucose uptake from fat and muscle cells, and glycogen synthesis in the liver (Cefalu et al., 2016). The overproduction of insulin by pancreatic β cells is the physiologic reaction to insulin resistance and can lead to decline of β cell function and, eventually to prediabetes and type 2 diabetes (Donath et al., 2005).The term "improving insulin sensitivity refers to the systemic responsiveness to glucose, which can be measured by

1. The insulin sensitivity index-measures the ability of endogenous insulin to lower glucose in extracellular fluids by inhibiting glucose release from the liver and stimulating the peripheral consumption of glucose, and.
2. The glucose-clamp technique, which measures the effect of changes in insulin concentration on glucose clearance-glucose uptake rate divided by plasma glucose concentration per unit of body surface area.

"Prediabetes" is the precursor stage before diabetes mellitus in which blood sugar is abnormally high (e.g. 100-125 mg/dL).

Impaired fasting glycemia and impaired glucose tolerance are two aspects of prediabetes that are similar in clinical definition (glucose levels too high for their context) but are physiologically distinct. Insulin resistance, metabolic syndrome (or syndrome X), and prediabetes are closely related to one another and have overlapping aspects.

"Anti-diabetic treatment" for type 2 diabetes include :

- **Metformin.** Generally, metformin is the first medication prescribed for type 2 diabetes. It works by improving the sensitivity of your body tissues to insulin so that your body uses insulin more effectively. Metformin also lowers glucose production in the liver. Metformin may not lower blood sugar enough on its own.
- **DPP-4 inhibitors.** These medications also reduce blood sugar levels. They do not cause weight gain. Examples of these medications are sitagliptin, saxagliptin, vildagliptin and linagliptin.
- **Sulfonylureas.** These medications help the body secrete more insulin. Examples of medications in this class include glyburide, glipizide, and glimepiride. Possible side effects include low blood sugar and weight gain.

- **Thiazolidinediones.** Like metformin, these medications make the body's tissues more sensitive to insulin. This class of medications has been linked to weight gain and other more-serious side effects, such as an increased risk of heart failure and fractures. Because of these risks, these medications generally are not a first-choice treatment. pioglitazone is an examples of thiazolidinediones.
- **GLP-1 receptor agonists.** These medications slow digestion and help lower blood sugar levels. Their use is often associated with some weight loss. This class of medications isn't recommended for use by itself.

Exenatide and liraglutide are examples of GLP-1 receptor agonists.

- **SGLT2 inhibitors.** These are the newest diabetes drugs on the market. They work by preventing the kidneys from reabsorbing sugar into the blood. Instead, the sugar is excreted in the urine.

Examples include canagliflozin and dapagliflozin.

- **Insulin therapy.** Some people who have type 2 diabetes need insulin therapy as well. In the past, insulin therapy was used as a last resort, but today it is often prescribed sooner because of its benefits.

Because normal digestion interferes with insulin taken by mouth, insulin must be injected. Depending on needs, a mixture of insulin types may be used throughout the day and night. Often, people with type 2 diabetes start insulin use with one long-acting shot at night.

Insulin injections involve using a fine needle and syringe or an insulin pen injector - a device that looks similar to an ink pen, except the cartridge is filled with insulin.

There are many types of insulin, and they each work in a different way. Examples include: insulin glulisine, insulin lispro, insulin aspart , insulin glargine, insulin detemir, insulin isophane.

## EXAMPLES

Hereinafter, the present invention is described in more details and specifically with reference to the examples.

**Example 1: Bimagrumab improves body composition and insulin sensitivity in insulin-resistant subjects**

**Study Design.** Study CBYM338X2206 was an exploratory phase Ib, randomized, double-blinded, placebo-controlled, single-center, single-dose study, conducted from 21-Aug-2012 to 25-Oct-2014 at the Profil Institute for Clinical Research, Inc., Chula Vista, CA. Sixteen healthy volunteers with insulin resistance were randomly assigned in a ratio of 10:6 to receive either a single dose i.v. infusion of 30 mg/kg bimagrumab or placebo over approximately 2 hours, followed by a 4-hour observation period. This single-dose treatment period was followed by a 24-week follow-up. All subjects were instructed to maintain their current (as of screening) level of physical activity and exercise behaviors throughout the study period. Primary endpoint was to determine the effect of bimagrumab on whole body insulin sensitivity and body composition. Patients were assessed at baseline and at 10 weeks (Day 71) after drug treatment and then followed up for 24 weeks. This clinical study was designed in accordance with the ICH Harmonized Tripartite Guidelines for Good Clinical Practice, with applicable local regulations, and with the ethical principles laid down in the Declaration of Helsinki. The study protocol and all amendments were reviewed by the independent Institutional Review Board (IRB) for the site of Sunrise, FL, 33323, USA.

**Population.** Adult male and female subjects of 18 to 65 years and who provided informed consent were included in the study. Other inclusion criteria were body mass index (BMI) of 18 to 45 kg/m$^2$ and a diagnosis of glucose intolerance, defined as blood glucose of 140-199 mg/dl at 2 hours after 75 g oral glucose administration (OGTT) or a homeostatic model assessment-insulin resistance (HOMA-IR) $\geq$ 2.6. The calculation is as follows: HOMA-IR = fasting glucose (in mg/dL) $\times$ fasting insulin (in mU/L) / 405. Key exclusion criteria were any disease, in particular diabetes, patients in testosterone, growth hormone, systemic glucocorticoids therapies and women of child-bearing potential.

**Hyperinsulinemic Euglycemic Clamp.** (Le et al, 2009) After receiving a standardized evening meal (10 kcal/kg; 50% carbohydrate, 30% fat, 20% protein), followed by an overnight fast of at least 12 hours, subjects were connected to a Biostator (Life Science Instruments, Elkhart, IN, USA). One hand was placed under a heating pad to allow sampling of arterialized venous blood. Insulin was infused intravenously for 180 minutes at a rate of 1 mU.kg-1.min-1 (Step 1) to maintain arterialized venous blood glucose at the target level of 90 mg/dL. After 180 minutes, the insulin infusion rate was increased to 2.5 mU.kg-1 min-1 for a further 180 minutes (Step 2), a steady-state hyperinsulinemia was sufficient to prevent hepatic glucose output. At the end of Step 2, the insulin infusion terminated. The infusion rate of 20% v/v glucose (Glucose Infusion Rate, or GIR) in mg.kg-1.min-1 required to keep blood glucose levels at 90 mg/dL was recorded throughout the euglycemic hyperinsulinemic clamp period (Steps 1 and 2), and especially during the last 30 minutes of both periods (at quasi steady-state). The GIR was used for the calculation of insulin sensitivity indices (i.e., *M, M1,* S1). The steady-state periods for insulin sensitivity measurements were defined as the time from 150 - 180 minutes in Steps 1 and 2. The total duration of the two-step H-E clamp procedures was approximately 7 hours. Blood samples for measurement of serum insulin and glucose concentrations were drawn at 0, 150, 160, 170, 180 min in Step 1 and 150, 160, 170, and 180 min following increase to the higher insulin infusion rate Step 2.

**IVGTT.** Following a minimum 8-hour overnight fast, subjects underwent an intravenous glucose tolerance test for assessment of first phase insulin release. Basal blood samples for glucose, and insulin were drawn at -5 and 0 minutes. At 0 minutes, a bolus of glucose 0.3 g/kg of body weight was administered as D50 (50% solution in sterile water). The delivery of the bolus was completed within 2 minutes. Blood samples were drawn at 2, 4, 6, 8, 10, 15, and 20 minutes, after which the cannula was removed.

**PET FDG-18/ CT scan for BAT assessment.** Positron emission tomography (PET) with radioactive [18]fluoro-desoxy-glucose (FDG) and associated with Computed Tomography (CT) were performed during "cold" exposure and during "warm" (or thermoneutral) conditions to all subjects at baseline and week 10 (Virtanen KA et al, 2009). BAT activation under cold exposure was achieved by cooling subjects with the subject, while wearing light clothing, spending 1 hour in a room adjacent to the scanner room, set at an ambient temperature of 17-18°C. During the PET/CT scan itself, cold exposure was maintained with the scanner room temperature set to 17-18°C. The scan obtained in warm conditions (temperature at 22-24°C) was performed either the day before or the day after the one obtained under cold conditions. All scans were obtained under overnight fasting conditions. Immediately following the shoulder area dynamic acquisition, the lower leg was scanned to image the gastrocnemius and soleus muscles as examples of fast and slow twitch muscle tissue. PET Images were quantified using the time-activity curves from each voxel and from the aorta using PMOD software giving an imaging of the body metabolic rate measurements in micromoles glucose/100g/min, within a 128 x 128 x 63 matrix, with expected values in the range of 5-15. By comparing PET scans obtained with "cold" and "warm" conditions, quantitative measures were extracted from each data set.

**DXA Scan.** (Albanese et al, 2003) All subjects had a whole body dual energy X-ray absorptiometry (DXA) scan, completed at baseline, week 6, week 10, week 14 and week 24 at a centralized scan analysis (Novartis CRO). All scans required the subjects to lie supine on the DXA table and a calibration phantom was used to ensure consistency in DXA readings. From these scans, absolute values and relative changes from baseline in lean body mass (LBM) and fat-body mass (FBM) were calculated. The exam took 1-2 min. The effective dose of a DXA whole body scan was 2.1 $\mu$Sv.

**Statistical Analysis.** Analysis of the data was performed under the direction of Novartis personnel. The pharmacodynamic (PD) variable (M/I) evaluated from the hyperinsulinemic euglycemic clamp was analyzed, endpoints of interest were change from baseline values, and these endpoints were analyzed separately. Log transformation was applied to each endpoint before analysis. The log-transformed ratio to baseline was analyzed by an ANCOVA model with treatment (bimagrumab and placebo) as fixed effects and log baseline value as covariate. The treatment estimates, contrasts of treatment difference (bimagrumab-placebo) and corresponding 90% confidence intervals were back transformed. Percent relative to baseline and placebo adjusted percent relative to baseline for treatment comparison along with corresponding 90% confidence interval were provided. Descriptive statistics of raw values, percent changes and absolute changes from baseline for the PD variable were provided by time point, treatment group and step. The AUC (area under curve) and Cmax (maximum value) were derived for glucose and insulin profiles, by IVGTT. DXA parameters and body weight data as long as HbA$_{1c}$, fasting insulin and glucose were analyzed separately using mixed effects model for repeated measurements. Endpoints of interest were all change from baseline values. The log-transformed ratio for DXA parameters and body weight, and the change from baseline were analyzed by an ANCOVA model with treatment (bimagrumab and placebo), visit and the interaction visit*treatment as fixed factors, log-baseline/baseline value as covariate and subject as random factors. The treatment estimates, contrasts of treatment difference (bimagrumab-placebo) and corresponding 90% confidence intervals were back transformed. Percent relative to baseline and placebo adjusted percent relative to baseline for treatment comparison along with corresponding 90% confidence interval were provided.

## RESULTS

**Trial Population.** The healthy volunteers that were enrolled in this study were predominantly Caucasian (94%), male (81%) and largely overweight or obese (mean BMI 29.3 kg/m$^2$, range: 21.1-37.7, median 28.6 kg/m$^2$). All subjects were insulin resistant as defined by a HOMA-IR $\geq$ 2.6 and were not diabetic; mean HbA$_{1c}$ were 5.49% ($\pm$0.39) and 5.35% ($\pm$0.24) in the bimagrumab and placebo group respectively. A total of 16 subjects were enrolled, 10 in the bimagrumab group and 6 in the placebo group. Both groups were comparable with respect to baseline characteristics with the exception of subjects in the placebo group being somewhat older with a mean age 47.3 years versus 42.2 year in the bimagrumab group. All baseline laboratory findings were comparable.

**Body composition changes.** A significant treatment effect with bimagrumab was observed on body composition at Week 10 as measured by dual energy X-ray absorptiometry (DXA) scan. As expected, the total lean body mass (LBM) increased with bimagrumab (mean of 2.7% at Week 10 [90% confidential interval (CI) 0.45, 5.01; p=0.049]) (Figure 1A), with a treatment effect that was observed as early as Week 6 (first post-dose scan) and was trending towards baseline by the end of the study (Week 24). Additionally, a significant reduction in total fat body mass (FBM) was observed at Week 10 in the bimagrumab group when compared to placebo, mean -7.9% [90% CI -12.5, -3; p=0.011]; this effect was observed as early as Week 6 (first post-dose scan) and persisted until the end of the study, mean of - 6.5% [90% CI -11.2, -1.5; p=0.034] at Week 24 (Figure 1B). Interestingly the effect of bimagrumab on LBM reached a plateau level after 6 weeks, with only an additional ~1% increase between Week-6 and Week-14, compared to the persistent decrease that was observed in FBM. There was no appreciable effect on body weight in either group throughout the study; the absolute increase of LBM with bimagrumab was mean 2 Kg ($\pm$1.4kg) compared to 750 g ($\pm$2.1 kg) in the placebo group at Week 10 compared to baseline, while the loss of absolute FBM was mean 2.3 Kg ($\pm$1.7 kg) with bimagrumab and 470 g ($\pm$0.7 kg) with placebo.

**Insulin sensitivity and HbA$_{1c}$.** Insulin sensitivity was measured by the 2 step hyperinsulinemic euglycemic clamp, and reported as the ratio of insulin sensitivity (*M*) adjusted for serum levels of insulin (*I*). The treatment effect of bimagrumab on insulin sensitivity (*M/I*) at Week 10 showed an increase by 21.8% [90% CI -8.9, 62.7; p=0.250] for Step 1 and 18.2% [90% CI -7.4, 50.9; p=0.247] for Step 2. Insulin sensitivity was also evaluated with the intravenous glucose tolerance test (IVGTT). The treatment effect of bimagrumab at Week 10 indicated a significant reduction in the insulin response to the glucose challenge with no treatment effect on glucose levels. The area under curve (AUC) for insulin was reduced by 38% [90% CI -52.6, -19; p=0.008] and the maximum concentration (Cmax) of insulin was reduced by 30.4% [90% CI -48.7, -5.6; p=0.056] (Figure 2). No differences were observed in fasting plasma glucose (p=0.674) in bimagrumab group compared to placebo group, while it was observed a significant reduction in the glucose AUC (10.3%, p=0.011) in the placebo group compared to bimagrumab group. A linear and significant relationship between loss of body fat mass and increase in insulin sensitivity at the hyperinsulinemic euglycemic clamp was observed (p=0.041 for Step 1, p=0.028 for Step 2), while no such association was observed between changes in lean body mass and insulin sensitivity (p=0.982 for Step 1, p=0.411 for Step 2) (Figure 3).The treatment effect of bimagrumab on HbA$_{1c}$ was seen as early as Week 6 and showed a reduction by 0.22% [90%CI -0.31, -0.12; p<0.001] that was sustained till the end of the follow-up at Week 24, mean -0.24% [90%CI -0.33, -0.14; p<0.001] (Figure 4). The placebo group showed a slow deterioration of HbA$_{1c}$ around Week 18, mean 0.11% [CI90% -0.01, 0.24; p=0.144], however not statistically significant.

**Brown adipose tissue (BAT).** The thermogenic capacity of BAT is defined by the difference in glucose metabolic rate ($\Delta$GMR) measured under neutral ambient temperature (i.e. "warm" 22-24°C) and at the end of a 90-min cold exposure (i.e. "cold" 17-18°C). Prior to treatment with bimagrumab, this cold challenge resulted in a GMR increase from $0.56\pm0.15$ $\mu$mol/100g/min to $0.67\pm0.24$ $\mu$mol/100g/min (+19%, N=15, p=0.12). At Week 10 after drug administration, the placebo group showed a trend through a decrease in the thermogenic response; such progression into further impairment of BAT activity was not observed in the bimagrumab group ($\Delta$GMR$_{[cold-warm]}$ placebo $-0.23\pm0.27$ $\mu$mol/min vs BYM338: 0. 06 $\pm0.35$ $\mu$mol/min, p=0.11). A trend for a reduction in BAT volume was also observed in the bimagrumab group at Week 10 while no effect on BAT volume was detected in the placebo group (BYM338: $-36.9\pm44.8$ mL vs placebo: $-4.7\pm18.8$ mL, p=0.12).

**Muscle strength.** The treatment effect of bimagrumab on muscle strength as measured by leg press showed a trend towards an increase by 33% [90% CI -5.5, 87.1; p=0.168] at Week 14 and by 45.3% [90% CI 3.3, 104.4; p=0.073] at the end of the study (Week 24). These effects did not reach statistical significance.

**Pharmacokinetics.** The pharmacokinetics of bimagrumab in overweight and obese individuals is consistent with that observed in lean and underweight people and exhibited a target-mediated drug disposition (TMDD) profile with a threshold for loss of the nonlinear clearance to be approximately at 10 $\mu$g/mL (Figure 5). As the loss of the nonlinear clearance could be linked to a loss of saturation of the receptors, a single dose of 30 mg/kg seemed to allow receptor saturation up to Week 10 (71 days), a time where treatment effects on body composition and insulin sensitivity were performed.

**Adverse events.** During this study, there were no serious adverse events (SAEs) and no adverse events (AEs) that led to study discontinuation. Acne, muscle spasms and myalgia were reported in 30% of bimagrumab-treated subjects and in none of the placebo-treated subjects. Diarrhea, muscle weakness and musculoskeletal stiffness were noted in 10% of the bimagrumab group and in none of the placebo group. Although upper respiratory tract infections were one of the most commonly reported AEs, they were not suspected to be related to study treatment. Anti-drug antibodies (ADAs) were detected in 2 out of 10 subjects following exposure to bimagrumab and were not neutralizing to either ActRIIA or ActRIIB. These ADAs were not associated with untoward safety findings or altered drug exposure.

## DISCUSSION

A single dose of bimagrumab led to a reduction in HbA$_{1c}$ and in insulin sensitivity of a magnitude that is considered clinically meaningful in insulin resistant, not diabetic individuals. These metabolic effects of bimagrumab were associated with profound changes in body composition, in the absence of an effect on body weight and of a change in lifestyle. The Look AHEAD trial demonstrated that weight loss of 5-10% of body weight at one year induces clinically significant improvements in cardiovascular risk factors and The Diabetes Prevention Program showed that 5% weight loss reduced the 5-year risk of developing new type 2 diabetes by 58% (Diabetes Prevention Program Research Group, 2002). A single dose of bimagrumab led to a significant increase in total LBM of 2.7% (2Kg $\pm$ 1.4) while the addition of exercise to a weight loss regimen with normal protein intake (1.2 g/kg/day) at best maintains baseline total LBM, and has not been shown to increase lean mass (Longland et al., 2016). Moreover bimagrumab led to a profound and lasting reduction in total FBM of 7.9% (2.3 Kg $\pm$ 1.7) at Week 10, a combined effect which, to our knowledge, was never observed with any other anti-obesity drug up to now. The net result of these changes in body composition ended in a neutral body weight effect which is better defined as a fitter body shape; yet the metabolic impact of these changes on glycemic parameters and on insulin sensitivity was significant. Bimagrumab has a novel mechanism of action that tackles the metabolic complications of obesity however further studies are needed in patients with obesity to evaluate whether a longer treatment period with bimagrumab islead to body weight loss in addition to improvements in body composition parameters, especially if combined with a calorie-restricted diet and adequate protein intake to support the anabolic effect of bimagrumab.

The improvement in $HbA_{1c}$ probably reflects a decrease in post-prandial glucose levels, as no effect was observed on fasting glucose levels. This may reflect an increase in peripheral insulin sensitivity, likely driven by an improvement at the level of the skeletal muscle. This treatment effect on $HbA_{1c}$ is higher than that obtained with metformin or lifestyle intervention ($HbA_{1c}$ down ~ 0.1% at 5 months) (Diabetes Prevention program Research Group, 2002) while it is comparable with that observed with liraglutide 3.0 mg qd in a similar population ($HbA_{1c}$ down 0.23% at Week 56) (Pi-Sunyer et al, 2015). The effect of bimagrumab on insulin sensitivity by the hyperinsulinemic euglycemic clamp and by the IVGTT, is on par with the effects of pioglitazone (45 mg) and liraglutide (1.8 mg) along with caloric restriction in a similar population, although the indices used slightly differ between the three studies (DeFronzo et al., 2011; Kim et al, 2013; Matsuda et al, 1999). Further and larger studies are needed in diabetic patients to evaluate whether treatment with bimagrumab isimprove insulin sensitivity in this population and istranslate to clinically meaningful reductions in $HbA_{1c}$.

Recent human studies have demonstrated how cold exposure and exercise can promote brown adipose tissue (BAT) activity (Ouellet et al., 2012; Dinas et al., 2014) and how BAT activity is typically impaired in overweight subjects (Van de Lans et al, 2014). Results from our study showed that the thermogenic capacity of BAT in obese and insulin resistant subjects tends to deteriorate over time and can be detected over just 4 weeks of time using FDG-PET/CT, moreover, such a worsening can be prevented by treating subjects with bimagrumab. This beneficial effect of ActRII blockade may be the result of an activation of mitochondrial oxidative metabolism in BAT as supported by preclinical data (Fournier et al 2012). Interestingly, preclinical studies have shown that not only intracellular triglycerides are the main fuel to sustain BAT energy metabolism but a cold challenge may also result in a near complete depletion of BAT lipids (Slocum et al., 2013; Ouellet et al, 2012, Cinti 2009, Baba et al 2010). This mechanism may then underlie the shrinkage of BAT volume also observed here in response to the bimagrumab treatment. Further investigation over a longer period of treatment with bimagrumab in combination with a physical activity program would be warranted to support this hypothesis. Overall bimagrumab was safe and well tolerated with no major safety findings in this new population of mainly overweight and obese individuals. Adverse events were minor and transient, as well as consistent with the previous clinical experience with bimagrumab, namely acne, particularly at dose levels used in this study, involuntary muscle contractions and myalgia. The biological explanation for acne remains unclear and is to be explored further.

The key findings from this study include profound positive changes in body composition in the absence of an effect on body weight. Treatment with bimagrumab resulted in a shift in body composition towards the "fit-fat" phenotype in the absence of exercise and diet. Furthermore, these shifts in body composition in the absence of weight loss mirrored the metabolic impact of weight loss on glycemic parameters.

Limitations of this trial include the fact that this is a pilot exploratory study with a small number of subjects; the population was heterogeneous for body mass index and included normal weight, overweight and obese individuals; evaluation of the effect size of bimagrumab on insulin sensitivity and $HbA_{1c}$ is limited because this population is in an insulin resistant state; this study did not evaluate the effect of bimagrumab on the various compartments of body fat, thus we cannot comment on effects on visceral, sub cutaneous and liver fat content. The mechanism for the observed improvement in insulin sensitivity has not yet been investigated; it could be secondary to reduced intra myocellular fat (Kuhlmann et al, 2003) content and/or to overall reduction in fat mass since it was observed a significant relation between FBM and insulin sensitivity ($M/I$) measured with the clamp, which was not observed with LBM (Figure 3). The extent to which hepatic insulin sensitivity is modified with bimagrumab is not known. We are speculating that the increase in lean mass could result in improved function and mobility in these individuals. The effect on muscle strength by 1-leg press is suggestive of a trend, though not significant and not conclusive. Repeat studies with a larger sample size are warranted to evaluate this question. In conclusion, bimagrumab may offer a novel approach for the treatment of metabolic complications of obesity such as insulin resistance. It is also possible that bimagrumab could be a novel insulin sensitizing therapeutic for type 2 diabetes through a meaningful effect on body composition. Unlike available therapeutics for type 2 diabetes, which tends to increase weight and fat mass, bimagrumab could reverse important features of the underlying pathophysiology of type 2 diabetes.

## Example 2 : Clinical study

### Study design

BYM338X2211 is a non-confirmatory, randomized, subject and investigator blinded, placebo-controlled, parallel arms study, investigating a 48-week treatment period with intravenous bimagrumab in overweight/obese patients with type 2 diabetes. Approximately 60 patients are enrolled and randomized. For patients who do consent for the optional MRI, their liver, visceral and subcutaneous fat content are assessed.

| Population | Approximately 60 obese patients (BMI:28-40 inclusive) with Type 2 Diabetes<br>Males and Females between the ages of 18-65 inclusive |
|---|---|
| Key Inclusion criteria | • Male and female, age 18 to 65 years (inclusive), in stable health condition as determined by past medical history, physical examination, vital signs, electrocardiogram, and laboratory tests at screening.<br>• Type 2 diabetes, with an HbA1c between 7% and 10% (inclusive) at screening, on metformin or DPP4 inhibitor agent monotherapy, with stable treatment for approximately 3 months prior to randomization.<br>• Body mass index (BMI) of 28 to 40 kg/m$^2$ (inclusive) at screening.<br>• Body weight between 65 and 140 kg (inclusive) at screening, and with a stable body weight ($\pm$5 kg) by history (patient report) and stable physical activity within 3 months prior to screening.<br>• At screening vital signs should be as follows: oral body temperature between 35.0-37.5 °C<br>• systolic blood pressure, 90 to 150 mm Hg<br>diastolic blood pressure, 50 to 90 mm Hg<br>pulse rate, 50 - 100 bpm |
| Key Exclusion criteria | • Pregnant or nursing (lactating) women, where pregnancy is defined as the state of a female after conception and until the termination of gestation confirmed by a positive hCG laboratory test.<br>• Women of child-bearing potential, defined as all women physiologically capable of becoming pregnant, unless they are using highly effective methods of contraception during dosing and for 6 months after stopping of investigational drug.<br>• Diabetes other than Type 2 such as Type 1 diabetes, surgically induced-diabetes; "brittle" type 2 diabetes as per investigator judgment, history of severe hypoglycemic epidoses in the year preceding screening, or hypoglycemic unawareness.<br>• History of clinically significant arrhythmias, unstable angina, myocardial infarction or stroke, coronary artery bypass graft surgery, or percutaneous coronary intervention (e.g. angioplasty or stent placement), within 6 months of screening or 1 year for drug-eluting stents.<br>• Use of any anti-obesity medications, nutritional supplements or over the counter products for weight loss within 3 months of screening. Use of medications known to induce weight gain such as some anti convulsant and psychotropic medications (e.g. clozapine) within 3 months of screening. |

### Screening (Days -21 to -8)

Participants undergo an onsite screening visit to determine their eligibility for the study. Subjects who qualify for enrollment following screening are scheduled for baseline assessments.

Planned lifestyle interventions (U.S. Department of Health and Human Services Food and Drug Administration Center for Drug Evaluation and Research 2008) include dietary counseling for weight loss with a daily caloric deficit of 500 kcal, with a diet that follows the American Diebetes Association (ADA) guidance for optimal glycemic control, and with protein intake of at least 1.2 g/kg/day to support muscle anabolism. Patients receive counseling for physical activity and are encouraged to follow the U.S Department of Health and Human Services (2008) guidelines (please refer to the SOM). These interventions are initiated at screening once eligibility is confirmed.

### Baseline (Days -7 to -1)

Prior to dosing (Day 1), patients who are eligible for enrollment following screening return to the clinic to undergo baseline assessments. To facilitate study conduct, patients may opt to be domiciled at Day -1 to complete baseline assessments prior to dosing on Day 1.

**Randomization and Dosing (Day 1)**

Eligible patients, based on screening and baseline assessments are randomized in a 1:1 ratio to receive either bimagrumab or placebo. Randomization are stratified according to baseline BMI into 2 strata:

- BMI between 28 kg/m$^2$ and 33kg/m$^2$ (inclusive) and
- BMI above 33 kg/m$^2$ and up to 40 kg/m$^2$ (inclusive).

Administration of bimagrumab or placebo is done via an intravenous infusion over 30 minutes followed by an observation period that includes safety and tolerability and PK sampling. Following all assessments, patients may be discharged from the Investigator site when the Investigator judges them to be medically stable, in good general health and not needing further observation.

**Treatment period (Days 1 - 336)**

Patients continue on background monotherapy as per eligibility criteria (see Inclusion criteria) throughout the study. All participants can continue to receive care for their diabetes and adaptation of their background therapy can be made.

Administration of bimagrumab or placebo is done via an intravenous infusion over 30 minutes followed by 1-hour observation period once every 4 weeks for a total of twelve doses. Bimagrumab is dosed based on body weight at 10 mg/kg, with a dose cap of 1200 mg for body weight equal to and above 120 kg. Placebo is provided as D5W, 5% Dextrose solution.

Patients receive regular monitoring and advice on diet and physical activity as part of their monthly site visits throughout the study.

Patients are asked to return to the Investigator site for dosing approximately every 4 weeks during the treatment period. During these visits, patients are evaluated for safety, tolerability, PK and efficacy.

The treatment period ends 4 weeks after the last administration (on Day 308/Week 44).

**Follow Up (Days 364 -392)**

After completion of the treatment period patients have a follow-up period of 8 weeks with regular monitoring for safety and efficacy (at Week 52) until the end of study visit (EOS) which take place 12 weeks after the last study drug administration .

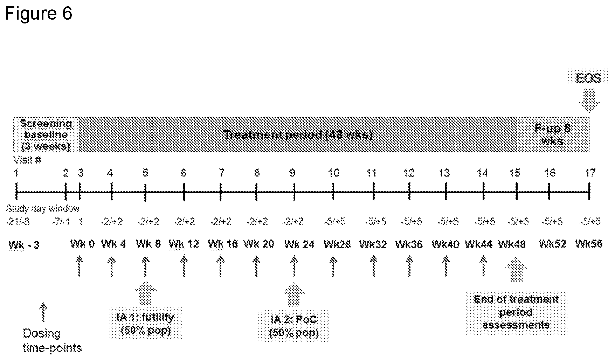

<u>**Rationale of study design**</u> (Figure 6)

The rationale for key elements of the study design include:

- **Randomization:** To decrease the chance of an imbalance in subject characteristics (e.g. age, BMI) between treatment groups.

- **Stratification:** BMI was selected as a stratification parameter as it is an important predictor of response on parameters of body composition/body weight and HbA1c (internal data). The expected median value for BMI in this patient population is 33 kg/m$^2$ (internal data), therefore 2 strata above and below/inclusive of the median ensure a balanced representation of subjects between placebo and active in each strata, i.e. strata of approximately similar size between the 2 arms.

- **Subject- and investigator-blinding:** To mitigate the risk of bias in treatment allocation, reporting and causality assessment of adverse events. Furthermore, this design decreases the potential confounding effect of intentional or unintentional behavioral changes made by subjects that are aware of their treatment assignment

- **Placebo arm:** The inclusion of the placebo provides a comparison group with bimagrumab and is included to allow for the double-blind design.

- **Lifestyle interventions:** Trials with anti-obesity agents have to demonstrate treatment benefits on body weight/-composition on a background of first line-therapy with lifestyle interventions. The daily caloric deficit of 500 Kcal is a standard approach and is expected to induce weight loss over the treatment period. It may also enhance the effect of bimagrumab on body composition and body weight. The American Diabetes Association (ADA) walking program is tailored to the type of population in this study and is a gentle, easy to implement approach for physical activity. Exercise is known to enhance the effect of bimagrumab on muscle function, which may support the treatment benefit of bimagrumab on body composition and weight.

- **Adequate protein intake:** this is important for optimal muscle maintenance/growth, the protein content of 1.2 g/kg/day is recommended to compensate for the caloric deficit.

**Standard of care diabetes therapy:** Patients remain on their standard of care treatment for diabetes, enabling the evaluation of added treatment benefit with bimagrumab on glycemic parameters. Oral diabetes monotherapy support selection of patients who are early in their disease state and thus without significant co-morbidities. Monotherapy is restricted to Metformin or DPP4 inhibitors, as these medications are less likely to affect body weight and thus confound the study results

**Rationale for dose/regimen, route of administration and duration of treatment**

**Dose rationale**

In healthy volunteers (HV) and sIBM patients, a 10 mg/kg dose of bimagrumab was shown to provide exposure levels (i.e. above 10 $\mu$g/mL) at which the anabolic effect is observed and maintained over dosing intervals of 4 weeks [CBYM338X2102 (N=6 subjects), CBYM338X2104 (N=47 subjects)], for up to six doses [CBYM338X2109 (N=35 subjects)] and up to one year [CBYM338B2203 (N=54 sIBM patients)]. The threshold for minimal target exposure for bimagrumab is approximately 10 $\mu$g/mL, a concentration below which nonlinear clearance is observed, suggesting loss of full receptor saturation and target-mediated drug disposition. In clinical studies to date, bimagrumab concentrations approximately at or above 10 $\mu$g/mL for at least 4 weeks in HV and more than one year in sIBM patients has been safe, well tolerated, demonstrated an increase in thigh muscle volume. The 26-week toxicology studies in Cynomolgus monkeys showed a chronic exposure at NOAEL (300 mg/kg/week) of approximately 300-fold and 55-fold for AUC and Cmax respectively when compared to human exposures at 10 mg/kg at steady state.

Dosing in this study is weight based for patients with body weight up to 120 kg, and is capped at 1200 mg for patients with body weight between 120 kg and 140 kg. Body-weight based dosing has proven to reduce variability in exposure in subjects/patients, and is implemented as applicable. Capped dose is selected for body weights > 120 kg because of the uncertainty of the effect of large body weight and body composition (% fat mass vs. % lean mass) on the pharmacokinetics, exposure, and safety profile of bimagrumab. To date, the pharmacokinetic data is limited in obese subjects, and the maximal body weight in dosed subjects has been 116 kg, in studies conducted with bimagrumab in overweight to obese subjects with insulin resistance (N=10), and obese healthy subjects (N=6). The maximal amount of bimagrumab administered to-date is 3500 mg (at a dose of 30 mg/kg), given i.v. and as a single dose for a maximal body weight of 116 kg. This dose did not show over-exposure and caused no safety concerns. A capped dose for these subjects is selected to avoid over-exposure and to maintain bimagrumab levels around the threshold for safe anabolic effects over 4-week dosing intervals. Specifically, the selected amount of 1200 mg translates to a body weight based dose ranging from 10 to 8.6 mg/kg for the body weight range of 120-140 kg, which is predicted to result in exposure levels within the safe and efficacious range for bimagrumab and with minimal risk of over-exposure.

**Rationale for duration of treatment**

A treatment duration of 48 weeks is selected to capture the temporal profile as well as maximal effect of bimagrumab on body fat mass. While a ceiling effect is typically observed on lean mass gain with bimagrumab, the loss of fat mass does not seem to plateau over a period of 24 weeks and even up to 64 weeks (Internal data).

**Rationale for follow-up period**

The extended follow-up period of 8weeks is selected to monitor the durability of treatment effect of bimagrumab on body fat mass, lean mass and glycemic control off treatment. The EOS visit being performed 12 weeks after the last administration covers the wash-out period of bimagrumab exposure associated with anabolic effect (approximately 8 weeks).

Rationale for choice of comparator

A placebo isbe used as a comparator in this study based on the following rationale:

- A placebo comparator is needed to maintain the double blind design.
- The placebo group is not intended as a primary efficacy comparator to bimagrumab, as the change from baseline in the bimagrumab treated group is the primary determination of efficacy.

Rationale for choice of background therapy

This is an anti-obesity trial in patients with T2D with the primary goal being the effect of bimagrumab on body composition. While improvement in glycemic control may occur as a result of improved body composition, this trial is not primarily a diabetes trial. Thus, patients need to maintain their background T2D therapy throughout to avoid a deterioration in glycemic control. T2D treatment is restricted to monotherapy for homogeneity of the study population and to enable interpretability of the data. Monotherapy is restricted to classes with minimal effect on body weight, including metformin, a first line therapeutic agent, and DPP4 inhibitors. If improvement in glycemic control is observed during the study, reduction in anti-diabetic treatment is allowed to prevent hypoglycemia.

**Treatment**

**Investigational treatment and control drugs**

The investigational drug, BYM338 (bimagrumab) 150mg LIVI (liquid in vial), is prepared by Novartis and supplied to the Investigator site as open labeled bulk medication. Study drug preparation is detailed in a separate pharmacy manual. Placebo is a dextrose 5% (D5W) in water infusion supplied by the site.

**Table 1 Overview of study medication**

| Study drug name | Formulation | Appearance (e.g., approximate size, color, etc.) | Packaging | Provided by |
|---|---|---|---|---|
| BYM338 (bimagrumab) | liquid in vial | water infusion | open labeled bulk medication | Novartis |
| Placebo | liquid | water infusion | | site |

Study drugs must be received at the study site by a designated person, handled and stored safely and properly, and kept in a secure location to which only the Investigator and designated staff have access. Upon receipt, the study drugs should be stored according to the instructions specified on the drug labels. Storage conditions must be adequately monitored and appropriate temperature logs maintained as source data. Appropriate documentation of the patient specific dispensing process must be maintained. Bulk medication labels is in the local language, complies with the legal requirements of each country, and includes storage conditions for the drug but no information about the patient.

**Additional study treatment**

No additional treatment beyond investigational drug and control drug are included in this trial.

**Background therapy**

Metformin or DPP4 inhibitor are requested as background therapy for patients to be eligible in the study.

**Treatment arms**

Patients are assigned to one of the following 2 treatment arms in a ratio of 1:1 Study treatments are defined as:

- BYM338 (bimagrumab) 10 mg/kg up to maximum 1200 mg monthly (12 doses)
- Placebo monthly (12 doses)

Efficacy / Pharmacodynamics

Pharmacodynamic assessments are specified below, with the methods for assessment and recording specified in the Site Operations Manual (SOM). Assessments isbe performed/samples collected at defined time points.

Pharmacodynamic (PD) samples are obtained and evaluated in all patients.

1. Glucose control assessment

Fasting insulin and glucose

Fasting glucose and insulin are taken at different times.

HbA1c

HbA1c reflects average glucose concentrations over the past 3 months and therefore provides a useful index of the glycemic control of bimagrumab over that time period. It is a standard endpoint used to assess the glycemic efficacy of any anti-diabetic medication. HbA1c is a key glycemic parameter which correlates with reduction of risk of diabetic complications.

HOMA-IR

Patients undergo fasting insulin and glucose at screening to estimate the degree of insulin resistance using the homeostatic assessment model of insulin resistance (HOMA-IR) and inverse of the HOMA-IR.

QUICKI

QUICKI is being evaluated as it is a better estimate of insulin resistance than HOMA-IR in patients with diabetes and elevated fasting glucose levels, e.g. > 170 mg/dl (Yokoyama et al 2004). QUICKI is a derived value of insulin sensitivity index using fasting glucose and insulin levels and provide additional and complementary information to that obtained with HOMA-IR (Hrebicek et al 2002).

2. Imaging

DXA Scan

Dual energy X-ray absorptiometry (DXA) is used to assess changes in body composition, including total fat and lean body mass (FBM and LBM) and appendicular skeletal fat and muscle mass (aFBM and aLBM). DXA instruments use an x-ray source that generates and is split into two energies to measure bone mineral mass and soft tissue from which fat and fat-free mass (or lean body mass) are estimated. The exam is quick (~5-6 min), precise (0.5-1%) and non-invasive. DXA scanners have the precision required to detect changes in muscle mass as small as 5%.

Quality assurance is an important issue in the use of DXA scans to determine body composition. DXA instrument manufacturer and model should remain consistent and their calibration should be monitored throughout the study. Use of a standardized scan acquisition protocol and appropriate and unchanging scan acquisition and analysis software is essential to achieve consistent results. Likewise, because of variability in interpretation of the scans, it is important to utilize centralized scan analysis by experienced staff.

Data collection and processing is explained in the imaging charter written by the imaging CRO supporting the study.

MRI scan

Magnetic resonance imaging (MRI) is used to assess changes in the percentage of fat in the liver (%fat fraction or %FF), the visceral and subcutaneous adipose tissue volumes in the abdominal region, as well as the paravertebral muscle cross-sectional area and associated fat contents (both the inter-muscle adipose tissue-IMAT and muscle FF contents). All images are acquired in the axial plane by using an imaging pulse sequences optimized for water/fat separation and adapted to the MRI system capabilities.

As for DXA scans, MRI scans are sent to the imaging center for central reading and results remain blinded to the investigator, patient and sponsor until after the study has been completed and the database has been locked. However, medically significant incidental findings (e.g. tumor) not related to the study analyses can be disclosed to the investigator as appropriate to the medical care of the patient. Detailed information can be located in the Imaging manual.

3. Anthropometric measurements

- Height

- Body weight

- Waist circumference

- Hip circumference

- Waist to hip ratio

- Body mass index (BMI) is calculated (Body weight (kg)/ [Height (m)]$^2$)

4. Performance measurements of physical function

Exercise Program

ADA walking guidelines is encouraged. Please refer to the SOM for additional details.

Timed Chair Stand

The timed chair stand test resembles a component of the Short Physical Performance Battery (Patel et al 2014), which assesses a person's ability to rise from a chair without the use of the arms once and then multiple times consecutively. This test requires no advanced technology and can be administered within a clinic or similar sized space. A description of the chair stand test, including the list of equipment, set up and script of instructions are available in the SOM.

Hand-Grip Strength Test

The purpose of this test is to measure the maximum isometric strength of the hand and forearm muscles. As a general rule, people with strong hands tend to be strong elsewhere, so this test is often used as a general test of strength.

5. Patient reported outcome

PRO: Impact of Weight on Quality of Life-Lite

The Impact of Weight on Quality of Life-Lite (IWQOL-Lite) is a survey instrument that is used to quantitatively assess an individual's perception of how their weight affects their day-to-day life. This instrument is especially valuable to validate the effectiveness of the treatment for obesity using metrics that go beyond the physical measurements of weight loss.

DTSQ (Diabetes Treatment Satisfaction Questionnaire)

The Diabetes Treatment Satisfaction Questionnaire (DTSQ) was first developed in the early 1980s. It is now widely used, particularly in clinical trials, but also for routine clinical monitoring. It is available in more than 100 languages. The original DTSQ is now referred to as the **status** version (DTSQs) in order to distinguish it from the DTSQ **change** version (DTSQc) which has been developed to overcome potential ceiling effects (i.e. where respondents score maximum or near-maximum satisfaction at baseline and can show little or no improvement at follow-up).

Analysis of the primary variable(s)

The primary aim of the study is to assess the effect of bimagrumab on fat mass at Week 24 and Week 48 of the treatment period.

Variable(s)

The primary efficacy variable is the change from baseline in fat mass at Week 24 and at Week 48.

Statistical model, hypothesis, and method of analysis

The study design enables evaluation of efficacy based on the following dual criteria

1) statistical significance (superior treatment effect, 1-sided 10% level) in fat mass ; and
2) clinical relevance of the change in fat mass (estimated median treatment effect of 5% or more) . Weight loss of 5% has been shown to translate into clinical benefit in an overweight/obese population with T2D (Franz et al 2015). Fat mass loss of a similar magnitude to the weight loss is expected to translate into similar clinical benefits, such as on glycemic control in a similar population.

The randomization is stratified by BMI category (>=28 kg/m2 and <=33 kg/m2, >33 kg/m2 to <=40 kg/m2) in order to achieve an approximate balance of BMI distribution across the two treatment groups. The cutoff value of 33 kg/m2 represents the expected median BMI in that population (based on internal data), therefore the two randomization strata are expected to be of similar size. A minimum of 10 patients is targeted for enrollment in the smaller stratum to ensure accurate precision on the treatment effect in both strata.

A longitudinal model describing fat mass over time is used (time modeled as a continuous variable), using all of the data collected from both randomization strata and adjusting for baseline fat mass, treatment arm, baseline BMI, with a random intercept and a random slope. The change in fat mass at Week 24 and Week 48 is estimated from that model. As a supportive analysis, the proportion of patients reaching at least 5% fat loss at Week 24 and Week 48 is presented by treatment group.

Handling of missing values/censoring/discontinuations

The primary analysis model described above is valid under the assumption of data missing at random. If the dropout rate is greater than 10% in any arm, other analysis methods is used to assess the sensitivity of the results to different methods for missing data handling.

Sensitivity analyses

Other models may be used (such as pattern-mixture models) if the dropout rate is higher than expected, in order to assess sensitivity of the primary efficacy conclusions to missing data.

Analysis of secondary variable(s)

A secondary efficacy variable of particular interest is the change in HbA1c at Week 24 and Week 48.

Other parameters of glucose control and insulin sensitivity (fasting glucose and insulin, HOMA-IR, QUICKI) and anthropometric body measurements (body weight, BMI, waist circumference, waist-to-hip ratio and lean body mass (LBM) as measured by DXA) are other secondary efficacy variables.

Efficacy / Pharmacodynamics

The secondary variable of HbA1c is analyzed in a similar fashion to fat mass, to assess the statistical significance (superior treatment effect, 1-sided 10% level) of bimagrumab therapy on HbA1c, and the clinical relevance of this effect (median treatment effect of 0.5%). A model is used to describe HbA1c over time and the change in HbA1c at all timepoints of interest (including Week 48) is estimated from that model. The analysis considers observations censored after a change in background anti-diabetic medication or dose. This analysis is expected to be unbiased because adjustments for background medication/dose are based on observed data (HbA1c, FPG), making the censored data following the medication change likely missing at random (MAR). Other adjustments for background anti-diabetic medications may be considered in the model. As a supporting analysis for metabolic changes, summaries of increase (and decrease) in background anti-diabetic medication may be done. A change in background anti-diabetic medication is defined as a change in daily dose and/or the addition of a second agent.

REFERENCES

Apovian CM, Aronne LJ, Bessesen DH, et al (2015) Pharmacological management of obesity: an endocrine Society clinical practice guideline. J. Clin. Endocrinol. Metab. p. 342-62.

Akpan I., Goncalves M.D., Dhir R., Yin X., Pistilli E.E., Bogdanovich S., Khurana T.S., Ucran J., Lachey J. and Ahima R.S. (2009) The effects of a soluble activin type IIB receptor on obesity and insulin sensitivity. Int. J. Obes. (Lond); 33:1265-1273.

Albanese C.V., Diessel E. and Genant H.K. (2003) Clinical applications of body composition measurements using DXA. J. Clin. Densitom., vol. 6, no. 2, 75-85.

Baba S., Jacene H.A., Engles J.M., Honda H. and Wahl R.L. (2010) CT Hounsfield units of brown adipose tissue increase with activation: preclinical and clinical studies. J. Nucl. Med. (2):246-50.

Boden G. (1997) Role of fatty acids in the pathogenesis of insulin resistance and NIDDM. Diabetes. 46(1):3-10.

Breitbart A, Auger-Messier M, Molkentin JD, et al (2011) Myostatin from the heart: local and systemic actions in cardiac failure and muscle wasting. Am. J. Physiol. Heart Circ. Physiol. p. H1973-82.

Cannon, B., and Nedergaard, J. (2004). Brown adipose tissue: function and physiological significance. Physiol. Rev. 84, 277-359.

Cefalu W.T., Bakris G, Blonde L., Boulton A.J.M., D'Alessio D., Hill Golden S., De Groot M., Greene E.L., Hu F.B., LeRoith D., et al. (2016). Standards of medical care in diabetes. The journal of clinical and applied research and education Vol 39, Suppl. 1 p. 1-119.

Chaston TB, Dixon JB, O'Brien PE (2007) Changes in fat-free mass during significant weight loss: a systematic review. Int J Obes (Lond) p. 743-50.

Chmelo EA, Beavers DP, Lyles MF, et al (2016) Legacy effects of short-term intentional weight loss on total body and thigh composition in overweight and obese older adults. Nutr Diabetes p. e203.

DeFronzo R.A . (2004) Dysfunctional fat cells, lipotoxicity and type 2 diabetes. Int J Clin Pract Suppl.; (143):9-21.

DeFronzo R.A., Tripathy D., Schwenke D.C., Banerji M., Bray G.A., Buchanan T.A., Clement S.C., Henry R.R., Hodis H.N., Kitabchi A.E., et al.; ACT NOW Study. (2011) Pioglitazone for Diabetes Prevention in Impaired Glucose Tolerance. N. Engl. J. Med.; 364: 1104-1115.

Diabetes Prevention Program Research Group (2002). Reduction in the Incidence of Type 2 Diabetes with Lifestyle Intervention or Metformin. N. Engl. J. Med. 346, 393-403.

Dinas PC, Nikaki A, Jamurtas AZ, Prassopoulos V, Efthymiadou R, Koutedakis Y, Georgoulias P, Flouris AD. (2015). Association between habitual physical activity and brown adipose tissue activity in individuals undergoing PET-CT scan. Clin Endocrinol (Oxf). 82(1):147-54.

Donath M.Y., Ehses J.A., Maedler K., Schumann D.M., Ellingsgaard H., Eppler E. and Reinecke M. (2005). Mechanisms of $\beta$-Cell Death in Type 2 Diabetes. Diabetes, 54: S108-S113.

Dresner A., Laurent D, Marcucci M, Griffin M.E., Dufour S, Cline G.W., Slezak L.A., Andersen D.K., Hundal R.S., Rothman D.L., et al (1999) Effects of free fatty acids on glucose transport and IRS-1-associated phosphatidylinositol 3-kinase activity. J Clin. Invest. 103(2): 253-259.

Fournier B., Murray B., Gutzwiller S., Marcaletti S., Marcellin D., Bergling S., Brachat S., Persohn E., Pierrel E., Bombard F., et al. (2012) Blockade of the activin receptor IIb activates functional brown adipogenesis and thermogenesis by inducing mitochondrial oxidative metabolism. Mol. Cell Biol. 32:2871-2879.

Franz MJ, Boucher JL, Rutten-Ramos S, et al (2015) Lifestyle weight-loss intervention outcomes in overweight and obese adults with type 2 diabetes: A systematic review and meta-analysis of randomized clinical trials. J. Acad. Nutr. Diet. p. 1447-1463.

Garito T et al. (2017) Bimagrumab improves body composition and insulin sensitivity in insulin-resistant individuals. Diabetes Obes Metab p1- 9.

Guo T., Jou W., Chanturiya T., Portas J., Gavrilova O. and McPherron AC (2009) Myostatin inhibition in muscle, but not adipose tissue, decreases fat mass and improves insulin sensitivity. PLoS ONE; 4:1-10.

Heymsfield SB, Gonzalez MC, Shen W, et al (2014) Weight loss composition is one-fourth fat-free mass: a critical review and critique of this widely cited rule. Obes Rev p. 310-21.

Hrebicek J, Janout V, Malincíková J, et al (2002) Detection of insulin resistance by simple quantitative insulin sensitivity check index QUICKI for epidemiological assessment and prevention. J. Clin. Endocrinol. Metab. p. 144-7.

Jensen MD, Ryan DH, Apovian CM, et al (2014) 2013 AHA/ACC/TOS guideline for the management of overweight and obesity in adults: a report of the American College of Cardiology/American Heart Association Task Force on Practice Guidelines and The Obesity Society. Circulation p. S102-38.

Kim S.H., Abbasi F., Lamendola C., Liu A., Ariel D., Schaaf P., Grove K., Tomasso V., Ochoa H., Liu Y.V., et al. (2013). Benefits of liraglutide treatment in overweight and obese older individuals with prediabetes. Diabetes Care. 36(10):3276-82.

Knowler WC, Barrett-Connor E, Fowler SE, et al (2002) Reduction in the incidence of type 2 diabetes with lifestyle intervention or metformin. N. Engl. J. Med. p. 393-403.

Kuhlmann J., Neumann-Haefelin C., Belz U., Kalisch J., Juretschke H.P., Stein M., Kleinschmidt E., Kramer W., Herling A.W.(2003) Intramyocellular lipid and insulin resistance: a longitudinal in vivo 1H-spectroscopic study in Zucker diabetic fatty rats. Diabetes 52(1):138-44.

Le D.S., Brookshire T., Krakoff J. and Bunt JC. (2009) Repeatability and reproducibility of the hyperinsulinemic-euglycemic clamp and the tracer dilution technique in a controlled inpatient setting. Metabolism 58:304-310

Lee SJ, Reed LA, Davies MV, et al (2005) Regulation of muscle growth by multiple ligands signaling through activin type II receptors. Proc.Natl.Acad.Sci.U.S.A; 102 (50):18117-18122.

Lim SS, Vos T, Flaxman AD, et al (2012) A comparative risk assessment of burden of disease and injury attributable to 67 risk factors and risk factor clusters in 21 regions, 1990-2010: a systematic analysis for the Global Burden of Disease Study 2010. Lancet p. 2224-60.

Lee S.J. and McPherron A.C. (2001) Regulation of myostatin activity and muscle growth. Proc Natl Acad Sci USA. 98(16): 9306-11.

Longland T.M., Oikawa S.Y., Mitchell C.J., Devries M.C., Phillips S.M. 2016. Higher compared with lower dietary protein during an energy deficit combined with intense exercise promotes greater lean mass gain and fat mass loss: a randomized trial. Am J Clin Nutr. 103(3):738-46.

Matsuda M. and DeFronzo R.A. (1999) Insulin sensitivity indices obtained from oral glucose tolerance testing: comparison with the euglycemic insulin clamp. Diabetes Care. (9):1462-70.

Mechanick JI, Garber AJ, Handelsman Y, et al (2012) American Association of Clinical Endocrinologists' position statement on obesity and obesity medicine. Endocr Pract p. 642-8.

Ouellet V., Labbé S.M., Blondin D.P., Phoenix S., Guérin B., Haman F., Turcotte E.E., Richard D. and Carpentier A.C. (2012) Brown adipose tissue oxidative metabolism contributes to energy expenditure during acute cold exposure in humans. J. Clin. Invest. 122(2):545-52.

Ogden CL, Carroll MD, Kit BK, et al (2014) Prevalence of childhood and adult obesity in the United States, 2011-2012. JAMA p. 806-14.

Ogden C.L., Carroll M.D., Fryar C.D. and Flegal K.M. (2015) Prevalence of Obesity Among Adults and Youth: United States, 2011-2014. CDC NCHS Data brief. n219

Patel MS, Mohan D, Andersson YM, et al (2014) Phenotypic characteristics associated with reduced short physical performance battery score in COPD. Chest p.1016-24.

Perseghin G., Price T.B., Petersen K.F, Roden M., Cline G.W., Gerow K, Rothman D.L. and Shulman G.I. (1996) Increased Glucose Transport-Phosphorylation and Muscle Glycogen Synthesis after Exercise Training in Insulin-Resistant Subjects. N. Engl. J. Med. 335:1357-1362

Pi-Sunyer X., Astrup A., Fujioka K., Greenway F., Halpern A., Krempf M., Lau D.C., le Roux C.W., Violante Ortiz R., et al. (2015). A Randomized, Controlled Trial of 3.0 mg of Liraglutide in Weight Management. SCALE Obesity and Prediabetes NN8022-1839 Study Group. N. Engl. J. Med. 2;373(1):11-22.

Reaven, G.M., Hollenbeck, C., Jeng, C.-Y., Wu, M.S., and Chen, Y.-D. (1988). Measurement of plasma glucose, free fatty acid, lactate, and insulin for 24h in patients with NIDDM. Diabetes. 37:1020-1024.

Saito M., Okamatsu-Ogura Y., Matsushita M., Watanabe K., Yoneshiro T., Nio-Kobayashi J., Iwanaga T., Miyagawa M., Kameya T, Nakada K., Kawai Y. and Tsujisaki M. (2009). High Incidence of Metabolically Active Brown Adipose Tissue in Healthy Adult Humans Effects of Cold Exposure and Adiposity. Diabetes. 58(7): 1526-1531.

Silljé HH, de Boer RA (2010) Myostatin: an overlooked player in heart failure? Eur. J. Heart Fail. p. 420-2.

Slocum N., Durrant J.R., Bailey D., Yoon L., Jordan H., Barton J., Brown R.H., Clifton L., Milliken T., Harrington W., et al. (2013). Responses of brown adipose tissue to diet-induced obesity, exercise, dietary restriction and ephedrine treatment. Exp. Toxicol. Pathol. 65(5):549-57.

Stabin MG (2008) Radiopharmaceuticals for nuclear cardiology: radiation dosimetry, uncertainties, and risk. J. Nucl. Med. p. 1555-63.

Suzuki ST, Zhao B, Yang J (2008) Enhanced muscle by myostatin propeptide increases adipose tissue adiponectin, PPAR-alpha, and PPAR-gamma expressions. Biochem. Biophys. Res. Commun. p. 767-73.

Tomlinson B, Cruickshank JM, Hayes Y, et al (1990) Selective beta-adrenocceptor partial agonist effects of pindolol and xamoterol on skeletal muscle assessed by plasma creatinine kinase changes in healthy subjects. Brj J Pharmac; 30: 665-672.

Trendelenburg AU, Meyer A, Jacobi C, et al (2012) TAK -1/p38/nNFkB signaling inhibits myoblast differentiation by increasing levels of Activin A. Skeletal Muscle; 2(1):3.

U.S. Department of Health and Human Services (2008) 2008 physical activity guidelines for Americans.

U.S. Department of Health and Human Services Food and Drug Administration Center for Drug Evaluation and Research (CDER) (2008) Guidance for Industry Diabetes Mellitus: Developing Drugs and Therapeutic Biologics for Treatment and Prevention. p. 1-5.

Van der Lans A.A., Wierts R., Vosselman M.J., Schrauwen P., Brans B. and Van Marken Lichtenbelt W.D. (2014). Cold-activated brown adipose tissue in human adults: methodological issues. Am. J. Physiol. Regul. Integr. Comp. Physiol. 307(2):R103-13.

Van Marken Lichtenbelt W.D., Vanhommerig J.W., Smulders N.M., Drossaerts J.M.A.F.L., Kemerink G.J., Bouvy N.D., Schrauwen P. and Teule G.J.J. (2009) Cold-Activated Brown Adipose Tissue in Healthy Men . N. Engl. J. Med. 360:1500-1508

Virtanen, K.A., Lidell, M.E., Orava, J., Heglind, M., Westergren, R., Niemi, T., Taittonen, M., Laine, J., Savisto, N.J.,

# EP 3 558 361 B1

Enerba¨ck, S., and Nuutila, P. (2009). Functional brown adipose tissue in healthy adults. N. Engl. J. Med. 360, 1518-1525.

World Health Organization (2015) World Health Organization obesity and overweight fact sheet.

Xia Y, Schneyer AL (2009) The biology of activin: recent advances in structure, regulation and function. J. Endocrinol. p. 1-12.

Yokoyama H, Emoto M, Fujiwara S, et al (2004) Quantitative insulin sensitivity check index and the reciprocal index of homeostasis model assessment are useful indexes of insulin resistance in type 2 diabetic patients with wide range of fasting plasma glucose. J. Clin. Endocrinol. Metab. p. 1481

Yoneshiro, T., Aita, S., Matsushita, M., Okamatsu-Ogura, Y., Kameya, T., Kawai, Y., Miyagawa, M., Tsujisaki, M., and Saito, M. (2011). Age-related decrease in cold-activated brown adipose tissue and accumulation of body fat in healthy humans. Obesity (Silver Spring) 19, 1755-1760.

## Claims

1. An activin receptor antagonist for use in the treatment of obesity or overweight condition in a human subject, wherein the activin receptor antagonist is an anti-ActRII receptor antibody;

   wherein the anti-ActRII receptor antibody comprises a light chain of SEQ ID NO: 7 or a sequence having 95% identity with SEQ ID NO: 7; and a heavy chain of SEQ ID NO: 9 or a sequence having 95% identity with SEQ ID NO: 9; and
   wherein the anti-ActRII receptor antibody comprises a heavy chain variable domain comprising CDR1 of SEQ ID NO: 1, CDR2 of SEQ ID NO: 2 and CDR3 of SEQ ID NO: 3; and a light chain variable domain comprising CDR1 of SEQ ID NO: 4, CDR2 of SEQ ID NO: 5 and CDR3 of SEQ ID NO: 6.

2. The activin receptor antagonist for the use according to claim 1, wherein said subject has a BMI $\geq 30$ kg/m$^2$ or a BMI of $\geq 25$ kg/m$^2$ to $< 30$ kg/m$^2$.

3. The activin receptor antagonist for the use according to claim 1, wherein the treatment comprises treating an obesity-related or overweight condition-related comorbidity selected from the group of type 2 diabetes, glucose intolerance, prediabetes, insulin resistance, high triglycerides, physical impairment, osteoporosis, renal disease, obstructive sleep apnea, sexual hormones impairment, endocrine reproductive disorders such as polycystic ovary syndrome or male hypogonadism, osteoarthritis, gastrointestinal cancers, dyslipidaemia, hypertension, heart failure, coronary heart disease, stroke, gallstones, altered gonadal hormone profile.

4. The activin receptor antagonist for the use according to any one of claims 1-3, wherein the treatment comprises the treatment, reduction or prevention of type II diabetes.

5. An activin receptor antagonist for use in improving glycemic control in a human subject suffering from type II diabetes, wherein the activin receptor antagonist is an anti-ActRII receptor antibody;

   wherein the anti-ActRII receptor antibody comprises a light chain of SEQ ID NO: 7 or a sequence having 95% identity with SEQ ID NO: 7; and a heavy chain of SEQ ID NO: 9 or a sequence having 95% identity with SEQ ID NO: 9; and
   wherein the anti-ActRII receptor antibody comprises a heavy chain variable domain comprising CDR1 of SEQ ID NO: 1, CDR2 of SEQ ID NO: 2 and CDR3 of SEQ ID NO: 3; and a light chain variable domain comprising CDR1 of SEQ ID NO: 4, CDR2 of SEQ ID NO: 5 and CDR3 of SEQ ID NO: 6.

6. The activin receptor antagonist for the use according to claim 5, wherein improving glycemic control is achieved by improving insulin sensitivity.

7. The activin receptor antagonist for the use according to claim 5 or 6, wherein the subject is suffering from obesity or overweight condition.

8. The activin receptor antagonist for the use according to claim 7, wherein DTSQ score (Diabetes treatment satisfaction questionnaire score), or IWQOL/IWQOL-lite (Impact of Weight on Quality of Life) score or other patient reported outcome (PRO) related to obesity and/or diabetes are improved.

9. The activin receptor antagonist for the use according to any one of the preceding claims, wherein the anti-ActRII receptor antibody is bimagrumab.

10. The activin receptor antagonist for the use according to claim 9, wherein bimagrumab is administered at a dose of 3 mg/kg or 10 mg/kg.

11. The activin receptor antagonist for the use according to claim 10, wherein bimagrumab is administered every 4 weeks.

**Patentansprüche**

1. Aktivin-Rezeptor-Antagonist zur Verwendung bei der Behandlung von Adipositas oder Übergewicht bei einem menschlichen Individuum, wobei es sich bei dem Aktivin-Rezeptor-Antagonisten um einen Anti-ActRII-Rezeptor-Antikörper handelt;

   wobei der Anti-ActRII-Rezeptor-Antikörper eine leichte Kette unter SEQ ID NO: 7 oder eine Sequenz, die zu 95 % mit SEQ ID NO: 7 identisch ist, und eine schwere Kette unter SEQ ID NO: 9 oder eine Sequenz, die zu 95 % mit SEQ ID NO: 9 identisch ist, umfasst; und
   wobei der Anti-ActRII-Rezeptor-Antikörper eine variable Schwerkettendomäne, die CDR1 unter SEQ ID NO: 1, CDR2 unter SEQ ID NO: 2 und CDR3 unter SEQ ID NO: 3 umfasst, und eine variable Leichtkettendomäne, die CDR1 unter SEQ ID NO: 4, CDR2 unter SEQ ID NO: 5 und CDR3 unter SEQ ID NO: 6 umfasst, umfasst.

2. Aktivin-Rezeptor-Antagonist zur Verwendung nach Anspruch 1, wobei das Individuum einen BMI $\geq$ 30 kg/m$^2$ oder einen BMI von $\geq$ 25 kg/m$^2$ bis < 30 kg/m$^2$ aufweist.

3. Aktivin-Rezeptor-Antagonist zur Verwendung nach Anspruch 1, wobei die Behandlung Behandeln einer mit Adipositas oder mit Übergewicht verbundenen Komorbidität, die aus der Gruppe Typ-2-Diabetes, Glucose-Intoleranz, Prädiabetes, Insulinresistenz, hohe Triglyceride, körperliche Beeinträchtigung, Osteoporose, Nierenkrankheit, obstruktive Schlafapnoe, Beeinträchtigung der Sexualhormone, endokrine Fortpflanzungsstörungen wie Syndrom polyzystischer Ovarien oder männlicher Hypogonadismus, Osteoarthritis, Krebserkrankungen des Verdauungstrakts, Dyslipidämie, Bluthochdruck, Herzinsuffizienz, koronare Herzkrankheit, Schlaganfall, Gallensteine, verändertes Keimdrüsenhormonprofil ausgewählt ist, umfasst.

4. Aktivin-Rezeptor-Antagonist zur Verwendung nach einem der Ansprüche 1-3, wobei die Behandlung die Behandlung, Reduzierung oder Vorbeugung von Typ-II-Diabetes umfasst.

5. Aktivin-Rezeptor-Antagonist zur Verwendung beim Verbessern der Blutzuckerregulation bei einem menschlichen Individuum, das an Typ-II-Diabetes leidet, wobei es sich bei dem Aktivin-Rezeptor-Antagonisten um einen Anti-ActRII-Rezeptor-Antikörper handelt;

   wobei der Anti-ActRII-Rezeptor-Antikörper eine leichte Kette unter SEQ ID NO: 7 oder eine Sequenz, die zu 95 % mit SEQ ID NO: 7 identisch ist, und eine schwere Kette unter SEQ ID NO: 9 oder eine Sequenz, die zu 95 % mit SEQ ID NO: 9 identisch ist, umfasst; und
   wobei der Anti-ActRII-Rezeptor-Antikörper eine variable Schwerkettendomäne, die CDR1 unter SEQ ID NO: 1, CDR2 unter SEQ ID NO: 2 und CDR3 unter SEQ ID NO: 3 umfasst, und eine variable Leichtkettendomäne, die CDR1 unter SEQ ID NO: 4, CDR2 unter SEQ ID NO: 5 und CDR3 unter SEQ ID NO: 6 umfasst, umfasst.

6. Aktivin-Rezeptor-Antagonist zur Verwendung nach Anspruch 5, wobei das Verbessern der Blutzuckerregulation durch Verbessern der Insulinsensitivität erreicht wird.

7. Aktivin-Rezeptor-Antagonist zur Verwendung nach Anspruch 5 oder 6, wobei das Individuum an Adipositas oder Übergewicht leidet.

8. Aktivin-Rezeptor-Antagonist zur Verwendung nach Anspruch 7, wobei der DTSQ(Diabetes Treatment Satisfaction Questionnaire)-Score oder IWQOL/IWQOL-Lite(Impact of Weight on Quality of Life)-Score oder ein anderes vom Patienten berichtetes Resultat (Patient Reported Outcome, PRO) in Verbindung mit Adipositas und/oder Diabetes verbessert ist.

**9.** Aktivin-Rezeptor-Antagonist zur Verwendung nach einem der vorhergehenden Ansprüche, wobei es sich bei dem Anti-ActRII-Rezeptor-Antikörper um Bimagrumab handelt.

**10.** Aktivin-Rezeptor-Antagonist zur Verwendung nach Anspruch 9, wobei Bimagrumab in einer Dosis von 3 mg/kg oder 10 mg/kg verabreicht wird.

**11.** Aktivin-Rezeptor-Antagonist zur Verwendung nach Anspruch 10, wobei Bimagrumab alle 4 Wochen verabreicht wird.

**Revendications**

1. Antagoniste du récepteur de l'activine destiné à être utilisé dans le traitement de l'obésité ou du surpoids chez un sujet humain, l'antagoniste du récepteur de l'activine étant un anticorps anti-récepteur ActRII ;

   l'anticorps anti-récepteur ActRII comprenant une chaîne légère de SEQ ID NO : 7 ou une séquence présentant 95 % d'identité avec SEQ ID NO : 7 ; et une chaîne lourde de SEQ ID NO : 9 ou une séquence présentant 95 % d'identité avec SEQ ID NO : 9 ; et
   l'anticorps anti-récepteur ActRII comprenant un domaine variable de chaîne lourde comprenant CDR1 de SEQ ID NO : 1, CDR2 de SEQ ID NO : 2 et CDR3 de SEQ ID NO : 3) ; et un domaine variable de chaîne légère comprenant CDR1 de SEQ ID NO : 4, CDR2 de SEQ ID NO : 5 et CDR3 de SEQ ID NO : 6.

2. Antagoniste du récepteur de l'activine selon la revendication 1, ledit sujet présentant un IMC $\geq$ 30 kg/m$^2$ ou un IMC $\geq$ 25 kg/m$^2$ à < 30 kg/m$^2$.

3. Antagoniste du récepteur de l'activine selon la revendication 1, le traitement comprenant le traitement d'une comorbidité liée à l'obésité ou d'une comorbidité liée au surpoids, choisie parmi le diabète de type 2, l'intolérance au glucose, le prédiabète, l'insulinorésistance, un taux élevé de triglycérides, une déficience physique, l'ostéoporose, une maladie rénale, l'apnée du sommeil obstructive, des troubles des hormones sexuelles, des troubles endocriniens de la reproduction tels que le syndrome des ovaires polykystiques ou l'hypogonadisme masculin, l'arthrose, les cancers gastro-intestinaux, la dyslipidémie, l'hypertension, l'insuffisance cardiaque, une maladie coronarienne, un accident vasculaire cérébral, les calculs biliaires, une altération du profil hormonal gonadique.

4. Antagoniste du récepteur de l'activine selon l'une quelconque des revendications 1 à 3, le traitement comprenant le traitement, la réduction ou la prévention du diabète de type II.

5. Antagoniste du récepteur de l'activine destiné à être utilisé dans l'amélioration du contrôle glycémique chez un sujet humain souffrant de diabète de type II, l'antagoniste du récepteur de l'activine étant un anticorps anti-récepteur ActRII ;

   l'anticorps anti-récepteur ActRII comprenant une chaîne légère de SEQ ID NO : 7 ou une séquence présentant 95 % d'identité avec SEQ ID NO : 7 ; et une chaîne lourde de SEQ ID NO : 9 ou une séquence présentant 95 % d'identité avec SEQ ID NO : 9 ; et
   l'anticorps anti-récepteur ActRII comprenant un domaine variable de chaîne lourde comprenant CDR1 de SEQ ID NO : 1, CDR2 de SEQ ID NO : 2 et CDR3 de SEQ ID NO : 3) ; et un domaine variable de chaîne légère comprenant CDR1 de SEQ ID NO : 4, CDR2 de SEQ ID NO : 5 et CDR3 de SEQ ID NO : 6.

6. Antagoniste du récepteur de l'activine selon la revendication 5, l'amélioration du contrôle glycémique étant obtenue par une amélioration de l'insulinosensibilité.

7. Antagoniste du récepteur de l'activine selon la revendication 5 ou 6, le sujet souffrant d'obésité ou de surpoids.

8. Antagoniste du récepteur de l'activine selon la revendication 7, le score DTSQ (score du questionnaire de satisfaction du traitement du diabète), le score IWQOL/IWQOL-lite (impact du poids sur la qualité de vie) ou tout autre résultat rapporté par le patient (PRO) lié à l'obésité et/ou au diabète étant améliorés.

9. Antagoniste du récepteur de l'activine selon l'une quelconque des revendications précédentes, l'anticorps anti-récepteur ActRII étant le bimagrumab.

10. Antagoniste du récepteur de l'activine selon la revendication 9, le bimagrumab étant administré à une dose de 3 mg/kg ou de 10 mg/kg.

11. Antagoniste du récepteur de l'activine selon la revendication 10, le bimagrumab étant administré toutes les 4 semaines.

Figure 1A

*p<0.05    —●— Bimagrumab  - ▲ - Placebo

Figure 1B

*p<0.05; **p=0.01    —●— Bimagrumab  - ▲ - Placebo

Figure 2

AUC=-38% (90% CI -52.6, -19.0; p=0.008)
Cmax=-30.4% (90% CI -48.7, -5.6; p=0.056)

Figure 3

Fat Body Mass

Lean Body Mass

Figure 4

Figure 5

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013006437 A
- WO 2010144452 A

- WO 2010125003 A

**Non-patent literature cited in the description**

- **APOVIAN CM** ; **ARONNE LJ** ; **BESSESEN DH et al.** Pharmacological management of obesity: an endocrine Society clinical practice guideline.. *J. Clin. Endocrinol. Metab.*, 2015, 342-62
- **AKPAN I.** ; **GONCALVES M.D.** ; **DHIR R.** ; **YIN X.** ; **PISTILLI E.E.** ; **BOGDANOVICH S.** ; **KHURANA T.S.** ; **UCRAN J.** ; **LACHEY J.** ; **AHIMA R.S.** The effects of a soluble activin type IIB receptor on obesity and insulin sensitivity. *Int. J. Obes. (Lond);*, 2009, vol. 33, 1265-1273
- **ALBANESE C.V.** ; **DIESSEL E** ; **GENANT H.K.** Clinical applications of body composition measurements using DXA.. *J. Clin. Densitom.*, 2003, vol. 6 (2), 75-85
- **BABA S.** ; **JACENE H.A.** ; **ENGLES J.M.** ; **HONDA H.** ; **WAHL R.L.** CT Hounsfield units of brown adipose tissue increase with activation: preclinical and clinical studies.. *J. Nucl. Med.*, 2010, 246-50
- **BODEN G.** Role of fatty acids in the pathogenesis of insulin resistance and NIDDM.. *Diabetes.*, 1997, vol. 46 (1), 3-10
- **BREITBART A** ; **AUGER-MESSIER M** ; **MOLKENTIN JD et al.** Myostatin from the heart: local and systemic actions in cardiac failure and muscle wasting.. *Am. J. Physiol. Heart Circ. Physiol.*, 2011, H1973-82
- **CANNON, B.** ; **NEDERGAARD, J.** Brown adipose tissue: function and physiological significance.. *Physiol. Rev.*, 2004, vol. 84, 277-359
- **CEFALU W.T.** ; **BAKRIS G** ; **BLONDE L.** ; **BOULTON A.J.M.** ; **D'ALESSIO D.** ; **HILL GOLDEN S** ; **DE GROOT M.** ; **GREENE E.L.** ; **HU F.B.** ; **LEROITH D. et al.** Standards of medical care in diabetes.. *The journal of clinical and applied research and education*, 2016, vol. 39 (1), 1-119
- **CHASTON TB** ; **DIXON JB** ; **O'BRIEN PE.** Changes in fat-free mass during significant weight loss: a systematic review.. *Int J Obes (Lond)*, 2007, 743-50
- **CHMELO EA** ; **BEAVERS DP** ; **LYLES MF et al.** Legacy effects of short-term intentional weight loss on total body and thigh composition in overweight and obese older adults.. *Nutr Diabetes*, 2016, e203

- **DEFRONZO R.A .** Dysfunctional fat cells, lipotoxicity and type 2 diabetes.. *Int J Clin Pract Suppl.*, 2004, 9-21
- **DEFRONZO R.A.** ; **TRIPATHY D.** ; **SCHWENKE D.C.** ; **BANERJI M.** ; **BRAY G.A.** ; **BUCHANAN T.A.** ; **CLEMENT S.C.** ; **HENRY R.R.** ; **HODIS H.N.** ; **KITABCHI A.E. et al.** ACT NOW Study.. *Pioglitazone for Diabetes Prevention in Impaired Glucose Tolerance. N. Engl. J. Med.;*, 2011, vol. 364, 1104-1115
- Reduction in the Incidence of Type 2 Diabetes with Lifestyle Intervention or Metformin.. *N. Engl. J. Med.*, 2002, vol. 346, 393-403
- **DINAS PC** ; **NIKAKI A** ; **JAMURTAS AZ** ; **PRASSOPOULOS V** ; **EFTHYMIADOU R** ; **KOUTEDAKIS Y** ; **GEORGOULIAS P** ; **FLOURIS AD.** Association between habitual physical activity and brown adipose tissue activity in individuals undergoing PET-CT scan.. *Clin Endocrinol (Oxf)*., 2015, vol. 82 (1), 147-54
- **DONATH M.Y.** ; **EHSES J.A.** ; **MAEDLER K.** ; **SCHUMANN D.M.** ; **ELLINGSGAARD H.** ; **EPPLER E.** ; **REINECKE M.** Mechanisms of β-Cell Death in Type 2 Diabetes.. *Diabetes*, 2005, vol. 54, S108-S113
- **DRESNER A.** ; **LAURENT D** ; **MARCUCCI M** ; **GRIFFIN M.E.** ; **DUFOUR S** ; **CLINE G.W.** ; **SLEZAK L.A.** ; **ANDERSEN D.K.** ; **HUNDAL R.S.** ; **ROTHMAN D.L. et al.** Effects of free fatty acids on glucose transport and IRS-1-associated phosphatidylinositol 3-kinase activity.. *J Clin. Invest.*, 1999, vol. 103 (2), 253-259
- **FOURNIER B.** ; **MURRAY B.** ; **GUTZWILLER S.** ; **MARCALETTI S.** ; **MARCELLIN D.** ; **BERGLING S.** ; **BRACHAT S.** ; **PERSOHN E.** ; **PIERREL E.** ; **BOMBARD F. et al.** Blockade of the activin receptor IIb activates functional brown adipogenesis and thermogenesis by inducing mitochondrial oxidative metabolism.. *Mol. Cell Biol.*, 2012, vol. 32, 2871-2879

- **FRANZ MJ** ; **BOUCHER JL** ; **RUTTEN-RAMOS S et al.** Lifestyle weight-loss intervention outcomes in overweight and obese adults with type 2 diabetes: A systematic review and meta-analysis of randomized clinical trials.. *J. Acad. Nutr. Diet.*, 2015, 1447-1463
- **GARITO T et al.** Bimagrumab improves body composition and insulin sensitivity in insulin-resistant individuals.. *Diabetes Obes Metab*, 1-9
- **GUO T.** ; **JOU W.** ; **CHANTURIYA T.** ; **PORTAS J.** ; **GAVRILOVA O** ; **MCPHERRON AC**. Myostatin inhibition in muscle, but not adipose tissue, decreases fat mass and improves insulin sensitivity.. *PLoS ONE;*, 2009, vol. 4, 1-10
- **HEYMSFIELD SB** ; **GONZALEZ MC** ; **SHEN W et al.** Weight loss composition is one-fourth fat-free mass: a critical review and critique of this widely cited rule.. *Obes Rev*, 2014, 310-21
- **HREBICEK J** ; **JANOUT V** ; **MALINCÍKOVÁ J et al.** Detection of insulin resistance by simple quantitative insulin sensitivity check index QUICKI for epidemiological assessment and prevention.. *J. Clin. Endocrinol. Metab.*, 2002, 144-7
- **JENSEN MD** ; **RYAN DH** ; **APOVIAN CM et al.** 2013 AHA/ACC/TOS guideline for the management of overweight and obesity in adults: a report of the American College of Cardiology/American Heart Association Task Force on Practice Guidelines and The Obesity Society.. *Circulation*, 2014, S102-38
- **KIM S.H.** ; **ABBASI F.** ; **LAMENDOLA C.** ; **LIU A.** ; **ARIEL D.** ; **SCHAAF P.** ; **GROVE K.** ; **TOMASSO V.** ; **OCHOA H.** ; **LIU Y.V. et al.** Benefits of liraglutide treatment in overweight and obese older individuals with prediabetes.. *Diabetes Care.*, 2013, vol. 36 (10), 3276-82
- **KNOWLER WC** ; **BARRETT-CONNOR E** ; **FOWLER SE et al.** Reduction in the incidence of type 2 diabetes with lifestyle intervention or metformin.. *N. Engl. J. Med.*, 2002, 393-403
- **KUHLMANN J.** ; **NEUMANN-HAEFELIN C.** ; **BELZ U.** ; **KALISCH J.** ; **JURETSCHKE H.P.** ; **STEIN M.** ; **KLEINSCHMIDT E.** ; **KRAMER W.** ; **HERLING A.W.** Intramyocellular lipid and insulin resistance: a longitudinal in vivo 1H-spectroscopic study in Zucker diabetic fatty rats.. *Diabetes*, 2003, vol. 52 (1), 138-44
- **LE D.S.** ; **BROOKSHIRE T.** ; **KRAKOFF J.** ; **BUNT JC.** Repeatability and reproducibility of the hyperinsulinemic-euglycemic clamp and the tracer dilution technique in a controlled inpatient setting.. *Metabolism*, 2009, vol. 58, 304-310
- **LEE SJ** ; **REED LA** ; **DAVIES MV et al.** Regulation of muscle growth by multiple ligands signaling through activin type II receptors.. *Proc.Natl.Acad.Sci.U.S.A*, 2005, vol. 102 (50), 18117-18122
- **LIM SS** ; **VOS T** ; **FLAXMAN AD et al.** A comparative risk assessment of burden of disease and injury attributable to 67 risk factors and risk factor clusters in 21 regions, 1990-2010: a systematic analysis for the Global Burden of Disease Study 2010. *Lancet*, 2012, 2224-60
- **LEE S.J.** ; **MCPHERRON A.C.** Regulation of myostatin activity and muscle growth. *Proc Natl Acad Sci USA.*, 2001, vol. 98 (16), 9306-11
- **LONGLAND T.M.** ; **OIKAWA S.Y.** ; **MITCHELL C.J.** ; **DEVRIES M.C.** ; **PHILLIPS S.M.** Higher compared with lower dietary protein during an energy deficit combined with intense exercise promotes greater lean mass gain and fat mass loss: a randomized trial.. *Am J Clin Nutr.*, 2016, vol. 103 (3), 738-46
- **MATSUDA M.** ; **DEFRONZO R.A.** Insulin sensitivity indices obtained from oral glucose tolerance testing: comparison with the euglycemic insulin clamp.. *Diabetes Care.*, 1999, 1462-70
- **MECHANICK JI** ; **GARBER AJ** ; **HANDELSMAN Y et al.** American Association of Clinical Endocrinologists' position statement on obesity and obesity medicine.. *Endocr Pract*, 2012, 642-8
- **OUELLET V.** ; **LABBÉ S.M.** ; **BLONDIN D.P.** ; **PHOENIX S.** ; **GUÉRIN B.** ; **HAMAN F.** ; **TURCOTTE E.E.** ; **RICHARD D.** ; **CARPENTIER A.C.** Brown adipose tissue oxidative metabolism contributes to energy expenditure during acute cold exposure in humans.. *J. Clin. Invest.*, 2012, vol. 122 (2), 545-52
- **OGDEN CL** ; **CARROLL MD** ; **KIT BK et al.** Prevalence of childhood and adult obesity in the United States, 2011-2012.. *JAMA*, 2014, 806-14
- **OGDEN C.L.** ; **CARROLL M.D.** ; **FRYAR C.D.** ; **FLEGAL K.M.** Prevalence of Obesity Among Adults and Youth: United States, 2011-2014.. *CDC NCHS Data brief. n219*, 2015
- **PATEL MS** ; **MOHAN D** ; **ANDERSSON YM et al.** Phenotypic characteristics associated with reduced short physical performance battery score in COPD.. *Chest*, 2014, 1016-24
- **PERSEGHIN G.** ; **PRICE T.B.** ; **PETERSEN K.F** ; **RODEN M.** ; **CLINE G.W.** ; **GEROW K** ; **ROTHMAN D.L.** ; **SHULMAN G.I.** Increased Glucose Transport-Phosphorylation and Muscle Glycogen Synthesis after Exercise Training in Insulin-Resistant Subjects.. *N. Engl. J. Med.*, 1996, vol. 335, 1357-1362
- **PI-SUNYER X.** ; **ASTRUP A.** ; **FUJIOKA K.** ; **GREENWAY F.** ; **HALPERN A.** ; **KREMPF M.** ; **LAU D.C.** ; **LE ROUX C.W.** ; **VIOLANTE ORTIZ R. et al.** A Randomized, Controlled Trial of 3.0 mg of Liraglutide in Weight Management. SCALE Obesity and Prediabetes NN8022-1839 Study Group.. *N. Engl. J. Med.*, 2015, vol. 373 (1), 11-22

- **REAVEN, G.M.** ; **HOLLENBECK, C.** ; **JENG, C.-Y.** ; **WU, M.S.** ; **CHEN, Y.-D.** Measurement of plasma glucose, free fatty acid, lactate, and insulin for 24h in patients with NIDDM.. *Diabetes*, 1988, vol. 37, 1020-1024
- **SAITO M.** ; **OKAMATSU-OGURA Y.** ; **MATSUSHITA M** ; **WATANABE K.** ; **YONESHIRO T.** ; **NIO-KOBAYASHI J.** ; **IWANAGA T** ; **MIYAGAWA M.** ; **KAMEYA T** ; **NAKADA K.** High Incidence of Metabolically Active Brown Adipose Tissue in Healthy Adult Humans Effects of Cold Exposure and Adiposity.. *Diabetes*, 2009, vol. 58 (7), 1526-1531
- **SILLJÉ HH** ; **DE BOER RA**. Myostatin: an overlooked player in heart failure?. *Eur. J. Heart Fail.*, 2010, 420-2
- **SLOCUM N.** ; **DURRANT J.R.** ; **BAILEY D.** ; **YOON L.** ; **JORDAN H.** ; **BARTON J.** ; **BROWN R.H.** ; **CLIFTON L.** ; **MILLIKEN T.** ; **HARRINGTON W. et al.** Responses of brown adipose tissue to diet-induced obesity, exercise, dietary restriction and ephedrine treatment.. *Exp. Toxicol. Pathol.*, 2013, vol. 65 (5), 549-57
- **STABIN MG**. Radiopharmaceuticals for nuclear cardiology: radiation dosimetry, uncertainties, and risk.. *J. Nucl. Med.*, 2008, 1555-63
- **SUZUKI ST** ; **ZHAO B** ; **YANG J**. Enhanced muscle by myostatin propeptide increases adipose tissue adiponectin, PPAR-alpha, and PPAR-gamma expressions.. *Biochem. Biophys. Res. Commun.*, 2008, 767-73
- **TOMLINSON B** ; **CRUICKSHANK JM** ; **HAYES Y et al.** Selective beta-adrenocceptor partial agonist effects of pindolol and xamoterol on skeletal muscle assessed by plasma creatinine kinase changes in healthy subjects.. *Brj J Pharmac*, 1990, vol. 30, 665-672
- **TRENDELENBURG AU** ; **MEYER A** ; **JACOBI C et al.** TAK -1/p38/nNFkB signaling inhibits myoblast differentiation by increasing levels of Activin A.. *Skeletal Muscle*, 2012, vol. 2 (1), 3
- *2008 physical activity guidelines for Americans.*, 2008
- *Guidance for Industry Diabetes Mellitus: Developing Drugs and Therapeutic Biologics for Treatment and Prevention.*, 2008, 1-5
- **VAN DER LANS A.A.** ; **WIERTS R.** ; **VOSSELMAN M.J.** ; **SCHRAUWEN P.** ; **BRANS B.** ; **VAN MARKEN LICHTENBELT W.D.** Cold-activated brown adipose tissue in human adults: methodological issues.. *Am. J. Physiol. Regul. Integr. Comp. Physiol.*, 2014, vol. 307 (2), R103-13
- **VAN MARKEN LICHTENBELT W.D.** ; **VANHOM-MERIG J.W.** ; **SMULDERS N.M.** ; **DROSSAERTS J.M.A.F.L.** ; **KEMERINK G.J.** ; **BOUVY N.D.** ; **SCHRAUWEN P.** ; **TEULE G.J.J.** Cold-Activated Brown Adipose Tissue in Healthy Men .. *N. Engl. J. Med.*, 2009, vol. 360, 1500-1508
- **VIRTANEN, K.A.** ; **LIDELL, M.E.** ; **ORAVA, J.** ; **HEGLIND, M.** ; **WESTERGREN, R.** ; **NIEMI, T.** ; **TAITTONEN, M.** ; **LAINE, J.** ; **SAVISTO, N.J.** ; **ENERBA¨CK, S.** Functional brown adipose tissue in healthy adults.. *N. Engl. J. Med.*, 2009, vol. 360, 1518-1525
- *World Health Organization obesity and overweight fact sheet.*, 2015
- **XIA Y** ; **SCHNEYER AL**. The biology of activin: recent advances in structure, regulation and function.. *J. Endocrinol.*, 2009, 1-12
- **YOKOYAMA H** ; **EMOTO M** ; **FUJIWARA S et al.** Quantitative insulin sensitivity check index and the reciprocal index of homeostasis model assessment are useful indexes of insulin resistance in type 2 diabetic patients with wide range of fasting plasma glucose.. *J. Clin. Endocrinol. Metab.*, 2004, 1481
- **YONESHIRO, T.** ; **AITA, S.** ; **MATSUSHITA, M.** ; **OKAMATSU-OGURA, Y.** ; **KAMEYA, T.** ; **KAWAI, Y.** ; **MIYAGAWA, M.** ; **TSUJISAKI, M.** ; **SAITO, M.** Age-related decrease in cold-activated brown adipose tissue and accumulation of body fat in healthy humans.. *Obesity (Silver Spring)*, 2011, vol. 19, 1755-1760